# EUROPEAN PATENT APPLICATION

(11) **EP 4 063 483 A1**
(43) Date of publication of application: **28.09.2022**
(21) Application number: 20890876.4
(22) Date of filing: 16.11.2020
(51) Int. Cl.: C12N 1/13, C12N 1/21, C12N 15/31, C12P 21/02

(54) **MODIFIED CYANOBACTERIA, METHOD FOR MANUFACTURING MODIFIED CYANOBACTERIA, AND METHOD FOR MANUFACTURING PROTEIN**

(30) Priority: 21.11.2019 JP 2019210114
(71) Applicant: Panasonic Intellectual Property Management Co., Ltd., Osaka-shi, Osaka 540-6207 (JP)
(72) Inventor: KOJIMA, Seiji, Osaka-shi, Osaka 540-6207 (JP)
(74) Representative: Novagraaf International SA
(86) International application number: PCT/JP2020/042549
(87) International publication number: WO 2021/100640

(57) **Abstract**

A modified bacterium in which a function of a protein involved in binding between an outer membrane (5) and a cell wall (4) of cyanobacterium is suppressed or lost.

SEQUENCE LISTING

<110>  Panasonic Intellectual Property Management Co., Ltd.

<120>  ENGINEERED CYANOBACTERIA, METHOD FOR GENERATING ENGINEERED
       CYANOBACTERIA, AND METHOD FOR GENERATING PROTEIN

<130>  P1030099WO01

<150>  JP 2019-210114
<151>  2019-11-21

<160>  22

<170>  PatentIn version 3.5

<210>  1
<211>  630
<212>  PRT
<213>  Synechocystis PCC6803

<400>  1

Met Leu Lys Leu Ser Trp Lys Ser Leu Leu Val Ser Pro Ala Val Ile
1               5                   10                  15

Gly Ala Ala Leu Val Ala Gly Ala Ala Ser Ala Ala Pro Asp Asn Val
            20                  25                  30

Thr Asn Ala Gln Val Leu Asp Gln Leu Asp Gln Tyr Thr Ala Glu Gly
        35                  40                  45

Gln Ser Ser Ala Ile Asp Gln Val Thr Ser Val Ser Glu Leu Arg Asp
    50                  55                  60

Val Gln Pro Thr Ala Trp Ala Tyr Glu Ala Leu Lys Ser Leu Val Glu
65                  70                  75                  80

Arg Tyr Gly Cys Ile Val Gly Tyr Pro Asp Arg Thr Phe Arg Gly Asp
                85                  90                  95

Arg Ala Leu Ser Arg Trp Glu Phe Ala Ala Gly Leu Asn Ala Cys Met
            100                 105                 110

Asn Val Met Glu Arg Leu Ile Gln Glu Asn Val Ala Val Leu Arg Glu
        115                 120                 125

Asp Ile Asp Lys Leu Lys Arg Leu Met Gln Glu Phe Glu Ala Glu Leu
    130                 135                 140

Ala Ala Leu Gly Ala Arg Val Asp Asn Leu Glu Ala Arg Thr Ser Phe
145                 150                 155                 160

Leu Glu Asp His Gln Phe Ser Thr Thr Thr Lys Leu Thr Gly Glu Val
                165                 170                 175

Ile Phe Ala Pro Thr Ala Ile Phe Gly Thr Lys Lys Thr Leu Asn Asn
            180                 185                 190

Gln Thr Asp Asn Asn Gln Ala Val Phe Gln Asn Arg Val Arg Leu Gln
        195                 200                 205

Phe Asn Thr Ser Phe Ser Gly Glu Asp Leu Leu Val Thr Arg Leu Ala
    210                 215                 220

Ala Gly Asn Gly Asn Arg Phe Lys Ser Phe Tyr Pro Thr Gln Asn Val
225                 230                 235                 240

Ile Asp Pro Glu Thr Gly Asn Pro Ile Gly Ile Val Gly Asp Tyr Tyr
                245                 250                 255

Glu Ser Pro Thr Phe Thr Gln Val His Gln Leu Ser Pro Gly Asp Asn
            260                 265                 270

Asn Asn Val Ala Val Asp Trp Leu Ala Tyr Tyr Val Pro Leu Asp Leu
        275                 280                 285

Gly Glu Asn Phe Arg Leu Asn Asn Tyr Ile Ala Ala Trp Gly Gly Ile
    290                 295                 300

Trp Asp Asp Phe Val Pro Thr Leu Asn Pro Tyr Phe Asp Asp Tyr Thr
305                 310                 315                 320

Gly Gly Lys Gly Ser Leu Ser Gln Phe Thr Ala Gln Asn Pro Ile Tyr
                325                 330                 335

Ser Ile Gly Gly Gly Thr Gly Ile Gly Thr Ser Leu Glu Leu Gly Phe
            340                 345                 350

Leu Ser Asn Leu Leu Gly Pro Thr Ser Leu Ser Leu Gly Tyr Leu Ala
        355                 360                 365

Ser Thr Gly Asn Asn Pro Ser Ser Gly Gly Ser Val Thr Asn Pro Ala
    370                 375                 380

Thr Gly Asn Asn Tyr Asp Phe Ser Ser Gly Gly Asn Gly Leu Phe Asn
385                 390                 395                 400

Gly Gly Tyr Ser Ala Leu Ala Gln Ile Thr Thr Asn Ile Phe Asp Arg
                405                 410                 415

Val Ser Leu Gly Phe Thr Tyr Val Asn Ala Tyr Thr Thr Pro Asp Ala
            420                 425                 430

Ala Ile Phe Gly Lys Gly Gly Thr Gln Gly Ile Val Gly Thr Thr Ala
        435                 440                 445

Ala Asn Leu Asn Arg Ser Glu Leu Asn Asp Asp Phe Val Asn Gly Leu
    450                 455                 460

Gly Val Gly Ala Pro Pro Val Asp Ala Ala Thr Tyr Asn Asn Asn Val
465                 470                 475                 480

Ser Gly Gly Ser Thr Ser Gly Asn Val Ile Asn Pro Tyr Asp Phe Gly
                485                 490                 495

Gly Lys Gln Thr Asn Ser Tyr Gly Val Gln Met Ala Trp Asn Ile Ala
            500                 505                 510

Asp Trp Leu Ser Phe Ser Ala Tyr Gly Ser Tyr Thr Asn Val Thr Leu
        515                 520                 525

Ile Gly Lys Asp Asn Gly Asp Ile Trp Thr Tyr Gly Gly Gly Phe Ala
    530                 535                 540

Phe Pro Asp Leu Gly Lys Glu Gly Asn Val Leu Gly Ile Phe Ala Gly
545                 550                 555                 560

Val Gln Pro Tyr Val Ser Gly Phe Thr Asn Asn Thr Leu Gly Thr Thr
                565                 570                 575

Phe Val Thr Thr Ala Asn Pro Leu Gln Val Glu Leu Phe Tyr Lys Tyr
            580                 585                 590

Gln Leu Thr Asp Asn Leu Ser Ile Thr Pro Gly Val Ile Trp Ile Ser
        595                 600                 605

Lys Pro Glu Gln Thr Thr Asn Ala Thr Asp Ala Phe Ile Gly Thr Val
    610                 615                 620

Arg Gly Thr Phe Thr Phe
625                 630

<210> 2
<211> 532
<212> PRT
<213> Synechococcus sp. NIES-970

<400> 2

Met Ser Lys Ala Met Arg Asn Leu Leu Ser Gly Ala Thr Val Ala Leu
1               5                   10                  15

Gly Ala Thr Val Ala Phe Ser Gly Ala Ala Ile Ala Glu Glu Asn Gln
            20                  25                  30

Ala Val Leu Asp Gln Ile Ser Gln Tyr Ser Thr Asp Asn Ser Val Ala
        35                  40                  45

Gln Val Asn Ser Val Phe Gln Leu Ser Asp Val Ser Pro Ser Asp Trp
    50                  55                  60

Ala Phe Asp Ala Leu Arg Asn Leu Val Glu Asn Tyr Asn Cys Ile Val
65                  70                  75                  80

Gly Tyr Pro Asp Gly Thr Phe Arg Gly Thr Arg Pro Leu Ser Arg Tyr
                85                  90                  95

Glu Phe Ala Ala Gly Leu Asn Ala Cys Leu Gln Gln Ile Glu Arg Met
            100                 105                 110

Leu Glu Gly Gly Gly Glu Val Thr Gln Glu Asp Leu Ala Ala Leu Arg
        115                 120                 125

Arg Leu Leu Asn Glu Phe Glu Ala Glu Leu Ala Thr Leu Gly Ala Arg
    130                 135                 140

Val Asp Asp Leu Asp Gly Arg Val Glu Phe Leu Glu Asp Asn Gln Phe
145                 150                 155                 160

Ser Thr Thr Thr Lys Leu Asn Gly Glu Val Ile Phe Asn Leu Gly Gly
                165                 170                 175

Ala Phe Gly Asp Ser Arg Ala Asp Gly Pro Gly Pro Ile Asp Asp Glu
            180                 185                 190

Phe Thr Phe Ser Asn Arg Val Arg Leu Asn Leu Asp Thr Ser Phe Thr
        195                 200                 205

Gly Arg Asp Arg Leu Arg Thr Arg Leu Glu Ala Gly Asn Ile Thr Arg
    210                 215                 220

Leu Asp Arg Ala Thr Gly Val Asp Ala Ala Arg Leu Gly Phe Asp Thr
225                 230                 235                 240

Asn Asn Gly Asn Asp Val Glu Ile Gly Lys Leu Phe Tyr Arg Phe Pro
                245                 250                 255

Val Gly Asp Asn Ile Thr Ala Tyr Val Gly Ala Val Gly Leu Gly Leu
            260                 265                 270

Asp Asp Ile Phe Asp Val Thr Asn Pro Tyr Phe Glu Ser Ser Gly Asn
        275                 280                 285

Gly Ala Leu Ser Arg Phe Gly Arg Phe Asn Pro Ile Tyr Arg Gly Pro
    290                 295                 300

Asp Gly Ala Gly Ala Gly Phe Asn Phe Asp Leu Gly Glu Arg Val Thr
305                 310                 315                 320

Phe Ser Ala Ala Tyr Leu Ala Asp Asp Gly Gly Ala Ser Ser Pro Val
                325                 330                 335

Ser Lys Asn Gly Leu Phe Asn Gly Asn Tyr Thr Ala Gly Ala Gln Leu
            340                 345                 350

Asp Phe Ala Pro Ser Asp Asn Ile Met Leu Ser Ala Val Tyr Thr Arg
        355                 360                 365

Arg Tyr Gln Gln Gly Gly Asp Val Asn Leu Ser Gly Ser Thr Gly Ser
    370                 375                 380

Asp Phe Ala Ala Asp Pro Phe Gly Phe Ala Ala Ser Ser Asn Asn Phe
385                 390                 395                 400

Gly Leu Gln Gly Ser Trp Leu Ile Gly Glu Lys Phe Asn Leu Gly Gly
                405                 410                 415

Trp Val Gly Tyr Thr Asn Ala Asp Leu Lys Asp Pro Val Val Thr Gly
            420                 425                 430

Asn Ala Asp Val Trp Asn Trp Ala Val Asn Ala Ala Leu Leu Asp Val
        435                 440                 445

Gly Gly Glu Gly Asn Thr Leu Gly Phe Ile Phe Gly Gln Pro Pro Lys
    450                 455                 460

Leu Thr Glu Ala Asp Ile Asp Phe Gly Gly Gly Pro Ile Thr Phe Asp
465                 470                 475                 480

Gly Ser Asp Ser Thr Tyr Leu Ala Glu Leu Gln Tyr Arg Tyr Lys Val
                485                 490                 495

Asn Asn Asn Ile Thr Ile Thr Pro Gly Ala Tyr Val Leu Trp Asn Pro

500                 505                 510

Asn Gln Asn Asp Ala Asn Asp Thr Ile Val Val Gly Thr Val Arg Thr
        515                 520                 525

Thr Phe Ser Phe
    530

<210>  3
<211>  567
<212>  PRT
<213>  Anabaena cylindrica PCC 7122

<400>  3

Met Ser Asn Leu Leu Trp Lys Ser Leu Val Val Ser Pro Ala Val Leu
1               5                   10                  15

Gly Ala Ala Leu Leu Val Ser Gly Thr Ala Ile Ala Ser Pro Ala Ala
            20                  25                  30

Gly Asn Ser Ala Ser Asn Ala Ala Thr Glu Val Ser Ile Leu Glu Thr
        35                  40                  45

Gln Ala Ala Thr Glu Val Val Gln Gln Pro Val Val Leu Ala Gln Val
    50                  55                  60

Lys Gln Asp Thr Gln Val Leu Asp Gln Val Lys Arg Tyr Ser Asn Glu
65                  70                  75                  80

Gly Gln Asn Leu Gln Ser Gln Val Thr Ser Val Ser Gln Phe Ser Asp
                85                  90                  95

Val Gln Pro Thr Asp Trp Ala Phe Gln Ala Leu Gln Ser Leu Val Glu
            100                 105                 110

Arg Tyr Gly Cys Ile Ala Gly Tyr Pro Asn Gly Thr Tyr Arg Gly Asn
        115                 120                 125

Arg Ala Leu Thr Arg Tyr Glu Phe Ala Ala Gly Leu Asn Ala Cys Leu
    130                 135                 140

Asp Arg Val Asn Glu Leu Ile Ala Thr Ala Thr Ala Asp Met Val Thr
145                 150                 155                 160

Lys Gln Asp Leu Ala Thr Leu Gln Arg Leu Gln Glu Glu Phe Ser Ala
                165                 170                 175

Glu Leu Ala Thr Leu Arg Gly Arg Val Asp Ala Leu Glu Ala Arg Thr

            180                 185                 190

Ala Glu Leu Glu Ala Asn Gln Phe Ser Thr Thr Thr Lys Leu Gln Gly
        195                 200                 205

Glu Ala Ile Phe Ala Leu Thr Asp Ala Phe Asn Gly Asp Ile Ala Gly
    210                 215                 220

Lys Thr Val Phe Gln Asp Arg Val Arg Leu Asn Leu Gln Ser Ser Phe
225                 230                 235                 240

Thr Gly Arg Asp Met Leu Asn Thr Arg Leu Ala Ala Gly Asn Ala Gln
                245                 250                 255

Asn Phe Gly Asn Thr Gly Glu Ser Arg Gln Thr Phe Asp Val Gly Ser
            260                 265                 270

Thr Gly Thr Asn Asn Val Val Leu Asp Lys Leu Thr Tyr Glu Ala Pro
        275                 280                 285

Val Gly Pro Ala Gln Val Tyr Ile Ala Ala Thr Gly Gly Lys His Ser
    290                 295                 300

His Tyr Ala Ala Val Asn Asn Pro Tyr Phe Phe Asp Lys Thr Asp Gly
305                 310                 315                 320

Gly Asn Gly Ala Leu Ser Thr Phe Ser Ser Glu Asn Pro Ile Tyr Arg
                325                 330                 335

Ile Gly Gly Gly Ser Gly Ile Ala Leu Asn Val Pro Phe Gly Gln Gly
            340                 345                 350

Gly Gly Ile Leu Lys Pro Ser Ser Leu Thr Leu Gly Tyr Leu Ala Ser
        355                 360                 365

Glu Ala Asn Ser Pro Ala Ala Gly Glu Gly Leu Phe Asp Gly Asn Tyr
    370                 375                 380

Ala Ala Leu Gly Gln Leu Asn Phe Asn Val Gly Asn Arg Ile Ala Leu
385                 390                 395                 400

Ala Ala Thr Tyr Val His Gly Tyr His Gly Ala Ser Ser Arg Leu Phe
                405                 410                 415

Asp Ser Gly Glu Gly Asp Asp Ile Val Gly Thr Ser Leu Ala Asn Asn
            420                 425                 430

Leu Ser Leu Gly Ser Ala Ser Ser Ser Asn Ser Tyr Gly Val Ser Ala
        435                 440                 445

Ala Phe Arg Pro Ser Asp Lys Leu Ser Val Ser Gly Phe Val Ser Tyr
    450                 455                 460

His Asp Ile Thr Gly Phe Gly Ala Gly Asp Asp Tyr Glu Ala Trp Ser
465                 470                 475                 480

Tyr Gly Val Gly Val Ala Leu Pro Asp Phe Gly Lys Lys Gly Asn Val
                485                 490                 495

Leu Gly Ile Phe Gly Gly Ala Gln Pro Tyr Ser Phe Asn Asn Asn Gly
            500                 505                 510

Gly Ala Asn Ser Gly Asp Ile Pro Tyr Gln Val Glu Gly Phe Tyr Lys
        515                 520                 525

Tyr Arg Val Ser Asp Asn Ile Ser Val Thr Pro Gly Val Ile Trp Leu
    530                 535                 540

Pro Ser Ile Gly Gln Ser Ser Ser Ser Glu Asp Thr Phe Ile Gly Thr
545                 550                 555                 560

Leu Arg Thr Thr Phe Thr Phe
                565

<210>  4
<211>  300
<212>  PRT
<213>  Synechocystis PCC6803

<400>  4

Met Arg Val Ile Leu Cys Gly Tyr Tyr Gly Gln Asp Asn Ala Gly Asp
1               5                   10                  15

Glu Ala Leu Leu Val Cys Leu Leu Gln Met Leu Pro Ala Thr Val Glu
            20                  25                  30

Pro Val Val Leu Ser Ala Asn Pro Gln Val Thr Thr Ala Arg Tyr Gly
        35                  40                  45

Val Lys Ala His Tyr Asn Arg Asp Trp Gly Lys Ile Trp Gln Leu Leu
    50                  55                  60

Gly Gln Cys Asp Gly Phe Ile Trp Gly Gly Gly Ser Leu Met Gln Asp
65                  70                  75                  80

Val Thr Ser Val Val Ser Pro Leu Tyr Tyr Gly Gly Leu Met Ala Ile
                85                  90                  95

Ala Gln Met Arg Arg Leu Lys Thr Ile Ala Trp Ala Gln Gly Ile Gly
            100                 105                 110

Pro Leu Arg Arg Pro Pro Leu Arg Trp Phe Thr Gln Gln Val Leu Arg
        115                 120                 125

Gly Cys Ser Gly Ile Ser Val Arg Asp Gln Ala Ser Leu Gln Leu Val
    130                 135                 140

Lys Gln Trp Gly Leu Lys Ala Phe Leu Ala Ala Asp Pro Val Trp Ser
145                 150                 155                 160

Leu Ala Ala Glu Asn Ala Ser Ser Ser Pro Ser Pro Leu Pro Met Val
                165                 170                 175

Ala Val Asn Leu Arg Ala His Pro Leu Leu Thr Leu Glu Arg Leu Ala
            180                 185                 190

Val Ile Thr Glu Ala Leu Arg Asp Phe Gln His Gln Thr Gln Thr His
        195                 200                 205

Val Arg Leu Ile Pro Leu Gln Lys Ser Gln Asp Leu Ala Ile Ala Glu
    210                 215                 220

Ala Ile Ala Val Glu Leu Pro Gly Ser His Glu Ile Leu Tyr Arg Pro
225                 230                 235                 240

Asp Pro Arg Glu Cys Lys Gly Leu Phe Arg Gly Ala Glu Phe Thr Ile
                245                 250                 255

Gly Met Arg Leu His Ser Leu Ile Met Ala Ala Ala Glu Gly Ser Ala
            260                 265                 270

Cys Phe Ala Leu Ser Tyr Asp Pro Lys Val Ser Arg Leu Ile Ala Glu
        275                 280                 285

Val Gly Leu Pro Gly Arg Glu Leu Ala Asp Leu Pro
    290                 295                 300

<210>  5
<211>  339
<212>  PRT
<213>  Synechocystis sp. PCC7942

<400>  5

Met Arg Ala Leu Leu Cys Gly Tyr Tyr Gly Glu Gly Asn Gly Gly Asp
1               5                   10                  15

Glu Ala Leu Leu Ala Ala Leu Leu Gln Leu Leu Pro Pro Thr Val Glu
            20                  25                  30

Pro Leu Val Leu Ser Gly Asn Pro Ala Ala Thr Arg Thr Cys Tyr Gly
        35                  40                  45

Val Glu Ala Cys Asp Arg Arg Ser Gly Ala Ala Val Trp Gln Ala Leu
    50                  55                  60

Arg Arg Ser Asp Leu Phe Ile Phe Gly Gly Gly Ser Leu Ile Gln Asp
65                  70                  75                  80

Val Thr Ser Trp Thr Ser Pro Leu Tyr Tyr Cys Gly Leu Met Gly Leu
                85                  90                  95

Ala Gln Gln Leu Gly Leu Lys Thr Ile Ala Trp Ala Gln Gly Ile Gly
            100                 105                 110

Pro Leu Gln Arg Lys Arg Thr Arg Trp Leu Ala Arg Arg Thr Phe Gln
        115                 120                 125

His Cys Asp Arg Ile Thr Val Arg Asp Arg Gly Ser Ala Ala Leu Leu
    130                 135                 140

Lys Glu Trp Gly Ile Ser Val Ala Ile Ala Pro Asp Pro Val Trp Ala
145                 150                 155                 160

Leu Glu Pro Leu Pro Leu Asp Gln Pro Leu Ser Asn Gln Glu Ala Ile
                165                 170                 175

Ala Val Cys Leu Arg Pro His Pro Glu Leu Thr Pro Glu Arg Ser Asp
            180                 185                 190

Arg Leu Lys Ala Ala Leu Thr Thr Leu Gln Ser Gln Thr Gln Ala Pro
        195                 200                 205

Ile Leu Leu Ile Pro Phe His His Gln Gln Asp Arg Pro Leu Ala Ala
    210                 215                 220

Gln Trp Ala Ser Glu Ile Pro Gln Ala Glu Val Leu Asp Trp Gln His
225                 230                 235                 240

Pro Arg Gln Leu Leu Ser Tyr Phe Gln Ser Val Arg Leu Thr Ile Ala
                245                 250                 255

Met Arg Phe His Gly Leu Val Met Ala Ala Ala Ala Gly Asn Arg Cys
            260                 265                 270

Phe Gly Leu Ser Tyr Asp Pro Lys Val Arg Arg Phe Leu Asp Ala Leu
        275                 280                 285

Glu Gln Pro Gly Trp Asp Leu Pro Glu Ile Pro Asp Ser Ala Glu Ala
    290                 295                 300

Ile Ala Ala Ala Trp Leu Glu Ser Trp Gln Ala Glu Pro Ile His Ala
305                 310                 315                 320

Ala Ala Ile Ala Asp Leu Arg Gln Gln Val Asp Val His Arg Gln Ala
                325                 330                 335

Leu Val Phe

<210>  6
<211>  350
<212>  PRT
<213>  Anabaena cylindrica PCC 7122

<400>  6

Met Val Lys Ile Arg Ala Leu Leu Ser Gly Tyr Tyr Gly Lys Gly Asn
1               5                   10                  15

Gly Gly Asp Glu Ala Leu Leu Ala Thr Leu Leu Gln Met Leu Pro Pro
            20                  25                  30

Asp Val Thr Pro Val Val Leu Ser Gly Asn Pro Glu Glu Thr Lys Lys
        35                  40                  45

Arg Tyr Gly Val Glu Ser Tyr Asn Arg Met Ala Phe Leu Gln Ile Ile
    50                  55                  60

Lys Ala Leu Arg Ser Cys Asp Ala Phe Ile Trp Gly Gly Gly Ser Leu
65                  70                  75                  80

Met Gln Asp Ala Thr Ser Ser Ile Ser Pro Phe Tyr Tyr Gly Gly Leu
                85                  90                  95

Met Ala Leu Ala Gln Ala Met Gly Leu Lys Thr Val Ala Trp Gly Gln
            100                 105                 110

Gly Ile Gly Pro Leu Leu Arg Ser Gln Thr Arg Trp Leu Ala Lys Arg
        115                 120                 125

Asn Phe Ala Leu Cys Thr Lys Ile Ser Val Arg Asp Arg Ser Ser Ala
    130                 135                 140

Ala Leu Leu Ser Asn Trp Gly Ile Pro His Ile Leu Ala Pro Asp Pro
145                 150                 155                 160

Val Trp Ala Leu Glu Ser Lys Pro Val Thr Glu Leu Ala Asp Leu Pro
                165                 170                 175

Lys Pro Arg Ile Ala Val Ile Leu Arg Asn His Pro Gln Leu Thr Glu
            180                 185                 190

Thr Arg Leu Ala Asn Leu Ile His Ala Leu Val Asp Leu Gln Gln Ala
        195                 200                 205

Thr Gln Ala Phe Ile Leu Leu Leu Pro Phe Gln Lys Ser Glu Asp Leu
    210                 215                 220

Asp Ile Ala Glu Lys Ile Gln Leu Gln Leu Lys Asp Val Ser Lys Ile
225                 230                 235                 240

Ile Cys Ala Glu Asp Pro Gln Leu Leu Lys Gly Ile Phe Arg Gly Val
                245                 250                 255

Glu Met Ala Ile Gly Met Arg Leu His Ser Leu Ile Met Ala Ala Ser
            260                 265                 270

Glu Gly Cys Arg Cys Phe Ala Leu Ser Tyr Asp Pro Lys Val Asn His
        275                 280                 285

Leu Met Glu Asp Leu Glu Ile Pro Gly Trp Asp Leu Lys Asp Leu Pro
    290                 295                 300

Thr Asp Val Asp Leu Met Ser Lys Thr Trp Ile Gln Tyr Tyr His Asn
305                 310                 315                 320

Ser Glu Ala Leu Ser Ser Glu Lys Ile Gln Ser Leu Val Asn Gly Ala
                325                 330                 335

Phe Leu His Arg Asp Leu Leu Arg Ala Ser Leu Ser Asn Lys
            340                 345                 350

<210>  7
<211>  1352
<212>  DNA
<213>  Synechocystis PCC6803

<400>  7

atgcttaaac tatcttggaa gtccctgtta gtgagccctg ctgtgatcgg tgccgccctt 60

gtggctggtg ctgctagtgc tgctcccgac aacgttacga atgctcaagt tttagaccaa 120

ttagaccaat acactgccga aggtcagtct tctgccattg atcaagtaac cagcgttagc 180

gaactccgcg atgtgcaacc cactgcctgg gcctatgaag cgctcaagag cttggtggaa 240

cgctacggtt gtatcgttgg ttaccctgac cgtaccttcc gtggtgatcg agccctcagc 300

cgttgggaat ttgccgctgg tcttaacgcc tgtatgaacg ttatggaacg tttgattcag 360

gaaaacgtgg ctgttctccg ggaggacatc gacaaactga aacgcctcat gcaggaattc 420

gaggcagaat tggctgcttt gggtgctcgg gttgataacc tcgaagcccg cacctctttc 480

ctcgaagacc atcagttttc taccaccacc aaattgaccg gggaagtaat atttgctccc 540

accgccattt ttggtaccaa aaagactctc aacaaccaaa ccgataacaa ccaagcggta 600

ttccagaacc gggttcgttt acagttcaac accagcttct ccggtgaaga cctgttggtc 660

actcgtctcg ccgctggtaa cggtaatcgc ttcaagagct tctatcctac tcagaatgtt 720

attgatcccg agaccggtaa ccccatcggt attgtaggtg actactatga aagccccacc 780

tttacccagg tgcaccagct ttcccccggc gacaacaaca acgtagccgt tgactggtta 840

gcttactatg ttcccttgga tctgggcgaa aacttccgtc tcaacaacta cattgctgct 900

tggggcggta tctgggatga cttcgttcct accctgaacc cctatttcga cgactacact 960

ggcggcaaag gttccttgag tcagttcacc gcccagaacc ccatctactc cattggtggc 1020

ggtactggta taggtacaag cctagagttg ggcttcctca gcaatctgtt gggccccact 1080

tccttgagct tgggttactt ggctagcacg ggcaacaacc ccagcagtgg tggttctgtt 1140

actaaccctg ccactggcaa caattatgac ttcagcagcg gtggtaacgg tttgtttaac 1200

ggtggctata gcgccttggc tcaaattacc accaacattt tcgaccgggt tagcctaggc 1260

ttcacctacg ttaatgccta caccacccct gatgccgcta tcttcggtaa aggtggcact 1320

caaggtatcg tcggtaccac tgcggctaac ct 1352

<210> 8
<211> 1353
<212> DNA
<213> Synechococcus sp. NIES-970

<400> 8
atgtctaaag caatgagaaa tcttctcagt ggtgcaaccg ttgctctcgg tgcaactgtt 60

gctttttctg gcgcagcgat cgccgaagaa aaccaagccg tcctcgacca gatcagtcaa 120

tacagcactg ataactccgt tgctcaggtc aacagcgttt tccagctcag cgacgtttcc 180

ccttccgatt gggcttttga cgcgctccgc aacttggttg aaaactacaa ctgtatcgtt 240

ggttatcctg atggtacttt ccgtggcact cgccccctca gccgttatga gttcgccgca 300

ggtctgaatg cttgtttgca acagatcgaa cggatgctcg aaggtggcgg tgaagtaacc 360

caagaagatc tcgcagccct ccgtcgtctg ctcaacgaat ttgaagcaga actagctacc 420

ctcggcgctc gtgttgatga ccttgatggc cgtgttgagt tccttgaaga caatcagttc 480

tccaccacca ccaaactgaa tggggaagtt atcttcaacc ttggtggcgc ttttggcgat 540

agcagagctg atggtcctgg cccaatcgat gatgagttca cattcagcaa tcgtgttcgt 600

ctgaacctag acactagctt cactggtcgc gatcgcctcc gcactcgtct tgaagctggc 660

aatatcactc gcttagaccg tgcaactggt gtagatgcag cccgtctcgg ctttgacact 720

aacaacggta atgatgttga aattgggaaa ttgttctacc gcttccctgt aggtgacaac 780

attactgcct atgtcggtgc cgttggccta ggtcttgatg acatatttga tgtaaccaac 840

ccatattttg agagcagcgg taatggtgcc ctctctcgtt tcggtcgttt taaccccatc 900

taccgtggcc ccgatggtgc tggtgctggt tttaacttcg atcttggtga aagagttacc 960

ttctccgcag cttaccttgc ggatgatggt ggtgctagca gccctgtctc gaagaatggc 1020

ctgttcaatg gcaactacac tgcgggtgct cagctcgatt ttgctccctc tgacaacatc 1080

atgctgtctg ccgtttatac ccgtcgctac caacagggtg gcgatgtcaa cctgagcggt 1140

agcactggta gtgattttgc tgcagatcct tttggttttg ctgcttcttc caacaacttc 1200

ggcttacaag gtagctggtt gatcggtgaa aaattcaacc ttggtggctg ggtcggctac 1260

accaatgctg atctcaaaga tcctgttgtc accggtaacg ctgatgtttg gaactgggct 1320

gttaacgccg ctctacttga tgtcggcggc gaa 1353

<210> 9
<211> 1360
<212> DNA
<213> Anabaena cylindrica PCC 7122

<400> 9
atgtctaatc tattgtggaa atccttggtg gttagcccag ccgtgttggg agccgcctta 60

ttagtttcag gaacagcgat cgcatcgccc gccgcaggca actcagcctc aaatgctgct 120

actgaagtat caatactaga aacacaagcg gcaacagagg ttgttcaaca acctgtagta 180

ttggctcaag tcaagcaaga cactcaggtt ttagatcaag ttaaacgcta cagtaacgaa 240

ggtcaaaatc tacaatctca agtaacatca gtctctcagt tttccgatgt ccaaccaact 300

gactgggcat tccaagcttt gcagtccttg gtggaacgtt atggttgtat cgcaggttat 360

cccaatggta catatcgcgg aaaccgtgct ttaacccgtt atgaatttgc cgctggtttg 420

aatgcctgtt tagaccgggt gaatgaattg attgccacag caacagctga catggtgaca 480

aaacaggatt tagccacctt acagcgcttg caagaagaat tttctgctga attggcaact 540

ttacgcggtc gtgtcgatgc tttggaagca cgcactgctg agttagaagc taatcaattc 600

tctactacca ccaaattgca aggtgaagct atttttgctc tgaccgatgc ttttaatggt 660

gacattgccg gtaagaccgt ttttcaagat agagtgcgtt tgaacctgca aagcagcttc 720

acaggtcgag atatgttgaa tacccgtttg gctgcgggta atgcacaaaa ctttggaaac 780

actggggaaa gtagacaaac ctttgacgtt ggtagcactg ggactaacaa tgttgtgtta 840

gacaaactga cctatgaagc tcctgttggt ccagcacagg tctacatagc agctacaggt 900

ggtaaacaca gccattatgc tgctgttaac aacccttact tctttgataa aactgatggt 960

ggtaacggtg ctttaagtac ctttagttct gaaaacccca tctatcgaat tggtggtggt 1020

tctggtattg cccttaatgt accctttggt caaggtggcg gtattctcaa accaagttca 1080

ttgacattgg gttatttggc ttctgaggct aatagtcctg ctgctggtga aggtttgttc 1140

gacggtaact atgctgcttt aggtcagtta aactttaacg ttggtaatcg cattgcctta 1200

gctgctactt atgtacatgg ttatcatggt gctagtagcc gtttgtttga ctcaggagag 1260

ggtgatgata tcgtaggtac atcactagct aataatctca gtttaggtag cgcctcgtcc 1320

agcaactcct acggtgtttc tgctgccttc agacctagcg 1360

<210> 10
<211> 1032
<212> DNA
<213> Synechocystis PCC6803

<400> 10
atgcgtgtca ttctgtgtgg ttactacggc caagacaatg ctggggatga agccctttta 60

gtctgtttgc tgcaaatgct tcctgcgacg gtggagccag tggtgctctc cgccaatccc 120

caggtcacca cagcgagata cggtgttaaa gcccactaca accgagactg gggcaaaatt 180

tggcagcttt tgggtcaatg tgacggtttt atctgggggg gaggcagttt gatgcaggat 240

gtgaccagcg tggttagtcc cctatactac ggtggtttga tggcgatcgc ccagatgcgg 300

cgtttaaaaa ccattgcctg ggcccagggc attggccccc ttagaaggcc gccgttgcgc 360

tggtttaccc aacaggtgct ccggggttgt agcggcatca gcgtacggga tcaagcttct 420

ttgcaattag ttaaacaatg gggcttaaag gcttttttag cggcggatcc ggtgtggtct 480

ttagccgctg aaaatgcgtc ttcttccccc agccctttgc ccatggtggc ggtgaatttg 540

cgggcccatc ctttgttaac cttggaacgc ttggcggtca ttaccgaagc cctaagggat 600

tttcaacacc agacccaaac ccatgtgcgg ctcattcccc tacaaaaatc ccaggatttg 660

gccattgccg aagcgatcgc cgtggaattg ccggggagtc atgaaattct ttaccgcccc 720

gaccccaggg aatgtaaagg tttgttccgg ggggcggaat tcaccattgg catgcggctc 780

cacagtttaa tcatggccgc ggcggagggc agtgcctgct ttgcccttag ttatgaccct 840

aaagttagtc gtttaatcgc agaggtggga ctaccgggcc gggaattggc ggatttaccc 900

agggatagca acgaactcag ccaggtttgg cagggacatt tccggcaaag acaaccctct 960

accggggtag agttcttgca aaaatctgcc cttcagcatc aaactttgtt gcagaaaatt 1020

tttgttgact ag 1032

<210> 11
<211> 1020
<212> DNA
<213> Synechocystis sp. PCC7942

<400> 11
atgcgggcgt tgctctgtgg ctattacgga gagggcaatg ggggagatga ggcgctgttg 60

gcagcgctgt tgcaactctt gccgccgaca gtggaaccgc tggtgctgtc ggggaatcct 120

gcggcaactc gcacttgcta cggcgtagaa gcttgcgatc gccgatcggg ggctgcggtt 180

tggcaggcac tgcgacgcag tgatctattc atctttggcg gcggcagcct gattcaagat 240

gtcacaagct ggacgagccc tctctactac tgcggtctga tggggctggc acaacagtta 300

ggtctcaaaa cgatcgcctg ggcccagggc attgggccac tgcagcgcaa acgaacccgc 360

tggttggcac gccgaacgtt tcagcactgc gatcgcatta cagtgcgcga tcgcggttct 420

gctgccttgc taaaagagtg ggggatttct gtggcgattg ccccggatcc cgtttgggct 480

ttagagccgc tgcctttaga tcagccgctg tcgaaccaag aggcgatcgc ggtttgtctg 540

cggccccacc ctgagttgac gcctgagcgc agcgatcgct tgaaagctgc cctgacaact 600

ctgcaatcgc aaacccaagc acccatcctg ctgattccct tccaccacca gcaagatcga 660

ccactggccg cgcaatgggc aagcgaaatt ccccaagcag aagttttgga ctggcagcat 720

ccgcgccagc tcctcagcta ctttcagtcg gtgcgattaa cgatcgccat gcgcttccat 780

ggattagtga tggcagcagc ggctggaaat cgttgctttg gcctcagcta tgaccccaaa 840

gtgcgccgtt tcctggatgc ccttgagcaa cccggctggg atttgccgga gattccagac 900

tctgctgagg cgatcgcggc cgcttggtta gagtcttggc aagcagaacc gattcatgcc 960

gctgcgatcg cggacctccg tcagcaagtg gatgtccatc gccaggcttt agttttttga 1020

<210> 12
<211> 1053
<212> DNA
<213> Anabaena cylindrica PCC 7122

<400> 12
ctacttatta ctcaatgatg ctctcaataa atcccgatgt aaaaaagctc catttactaa 60

agattgtatt ttctcagatg ataatgcttc gctattgtga taatattgaa tccaggtttt 120

actcattaaa tctacatcag ttggtaaatc ctttaaatcc catccaggta tttctaaatc 180

ttccatcaaa tggttaacct tggggtcata actcaaagca aaacaacgac aaccttcact 240

agctgccata attaaactgt gtagacgcat ccctattgcc atttctacac cacgaaatat 300

accttttaaa agttgtggat cttctgcaca gataatttta ctaacatctt ttaattgcag 360

ttgaattttt tctgcaatat ctaaatcttc acttttttga aaaggcaata ataaaataaa 420

tgcttgggta gcttgttgta aatcaactaa ggcatggatt aaatttgcta accgagtttc 480

tgtaagttgg ggatgatttc ttaaaataac ggcaattcta ggttttggta aatctgctaa 540

ttctgttact ggtttagatt ctaaagccca aactggatca ggtgcaagaa tatgaggtat 600

gccccaattt gataataaag ccgcactaga gcgatcgcgt acactgattt tagtacaaag 660

agcaaaattt cgctttgcta accaacgagt ttgcgatcgc aataaaggcc ctattccctg 720

tccccaagct acagttttca aacccatcgc ttgagctaaa gccattaatc ccccataata 780

aaaagggcta atgctgctag tcgcatcctg cattaaactt ccaccacccc agatgaaagc 840

atcacaggaa cgcaaagctt tgataatctg caaaaacgcc atacggttgt aactttccac 900

accataacgc tttttagttt cctcaggatt cccagaaagc accacaggcg ttacatcagg 960

tggtagcatt tgcagaagtg tcgccaacaa agcttcatca ccaccattac ctttaccgta 1020

atacccagac aataacgccc gtatcttcac cat 1053

<210> 13
<211> 40
<212> DNA
<213> Artificial Sequence

<220>
<223> Synthesized Primer

<400> 13
cagtgaattc gagctcggta tatagcgttg cagtccctgg 40

<210> 14
<211> 40
<212> DNA
<213> Artificial Sequence

<220>
<223> Synthesized Primer

<400> 14
gattacgcca agcttgcatg accgcggtca cttcataacc 40

<210> 15
<211> 47
<212> DNA
<213> Artificial Sequence

<220>
<223> Synthesized Primer

<400> 15
cagtgaattc gagctcggta ataaccgttg tcccttttgt ttcatcg 47

<210>  16
<211>  45
<212>  DNA
<213>  Artificial Sequence

<220>
<223>  Synthesized Primer

<400>  16
tgttagtgag ccctgctgtt agctcccagt atctctatca ctgat                    45


<210>  17
<211>  51
<212>  DNA
<213>  Artificial Sequence

<220>
<223>  Synthesized Primer

<400>  17
acagcagggc tcactaacag ttttagagct agaaatagca agttaaaata a             51


<210>  18
<211>  44
<212>  DNA
<213>  Artificial Sequence

<220>
<223>  Synthesized Primer

<400>  18
gattacgcca agcttgcatg gggaacaagc tgaatctggg catc                     44


<210>  19
<211>  45
<212>  DNA
<213>  Artificial Sequence

<220>
<223>  Synthesized Primer

<400>  19
ttttagtctg tttgctgcat agctcccagt atctctatca ctgat                    45


<210>  20
<211>  51
<212>  DNA
<213>  Artificial Sequence

<220>
<223>  Synthesized Primer

<400>  20
tgcagcaaac agactaaaag ttttagagct agaaatagca agttaaaata a             51


<210>  21
<211>  19

<212>  DNA
<213>  Synechocystis PCC6803

<400>  21
acagcagggc tcactaaca                                                    19


<210>  22
<211>  19
<212>  DNA
<213>  Synechocystis PCC6803

<400>  22
tgcagcaaac agactaaaa                                                    19

## Description

### [Technical Field]

The present disclosure relates to a modified cyanobacterium having improved secretory productivity of protein that is produced within the bacterial cell and secreted to the outside of the bacterial cell, a modified cyanobacterium production method, and a protein production method.

### [Background Art]

Bio-manufacturing schemes that do not depend on fossil fuel and have low environmental load have been desired for a wide range of industrial fields from chemical industry to agricultural, fishery, and livestock industry. Bio-manufacturing using microbes has received attention as production systems that satisfy the requirements described above, because it can be performed in an environment of ambient pressure and temperature and has become able to produce a wide range of compounds by recent development of genetic manipulation technology. Among others, photosynthetic microbes such as cyanobacterium and algae can utilize carbon dioxide (CO₂) in air as a carbon source with light as an energy source and are therefore particularly expected as carbon-neutral next-generation bio-manufacturing systems.

For example, ethanol (Non Patent Literature (NPL) 1), isobutanol (NPL 2), alkanes (Patent Literature (PTL) 2), fatty acid (PTL 1), and protein (NPL 3) have been reported as materials that are produced using cyanobacterium.

For example, NPL 4 discloses an approach for allowing cyanobacterium to express protein.

### [Citation List]

### [Patent Literature]

[PTL 1] Japanese Patent No. 6341676
[PTL 2] Japanese Unexamined Patent Application Publication No. 2015-109828

### [Non Patent Literature]

[NPL 1] Jason Dexter and Pengcheng Fu, "Metabolic engineering of cyanobacteria for ethanol production", Energy & Environmental Science, Royal Society of Chemistry, 2009, Vol.2, pp.857-864
[NPL 2] Shota Atsumi et al., "Direct photosynthetic recycling of carbon dioxide to isobutyraldehyde", Nature Biotechnology, Nature Publishing Group, 2009, Vol.27, No.12, pp.1177-1180
[NPL 3] Jie Zhou et al., "Discovery of a super-strong promoter enable efficient production of heterologous proteins in cyanobacteria", Scientific Reports, Nature Research, 2014, Vol.4, Article No.4500
[NPL 4] Andrew H. Ng et al., "Fine-Tuning of Photoautotrophic Protein Production by Combining Promoters and Neutral Sites in the Cyanobacterium Synechocystis sp. Strain PCC 6803", Applied and Environmental Microbiology, American Society for Microbiology, 2015, Vol.81, pp.6857-6863

### [Summary of Invention]

### [Technical Problem]

However, in the aforementioned conventional techniques, it is difficult to produce proteins efficiently using cyanobacteria.

In view of this, the present disclosure provides a modified cyanobacterium having improved protein secretory productivity, a modified cyanobacterium production method, and a protein production method using a modified cyanobacterium.

### [Solution to Problem]

A modified cyanobacterium according to an aspect of the present disclosure is a modified cyanobacterium in which a function of a protein involved in binding between an outer membrane and a cell wall of cyanobacterium is suppressed or lost.

### [Advantageous Effects of Invention]

The modified cyanobacterium and the modified cyanobacterium production method are capable of providing a modified cyanobacterium having improved protein secretory productivity. Furthermore, the protein production method according to the present disclosure is capable of efficiently producing protein.

### [Brief Description of Drawings]

[FIG. 1]

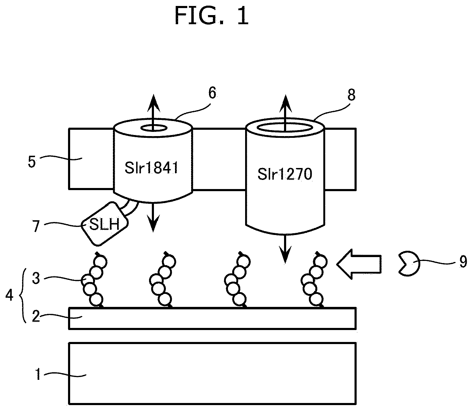

   FIG. 1 is a diagram schematically illustrating a cell surface of a cyanobacterium.
[FIG. 2]

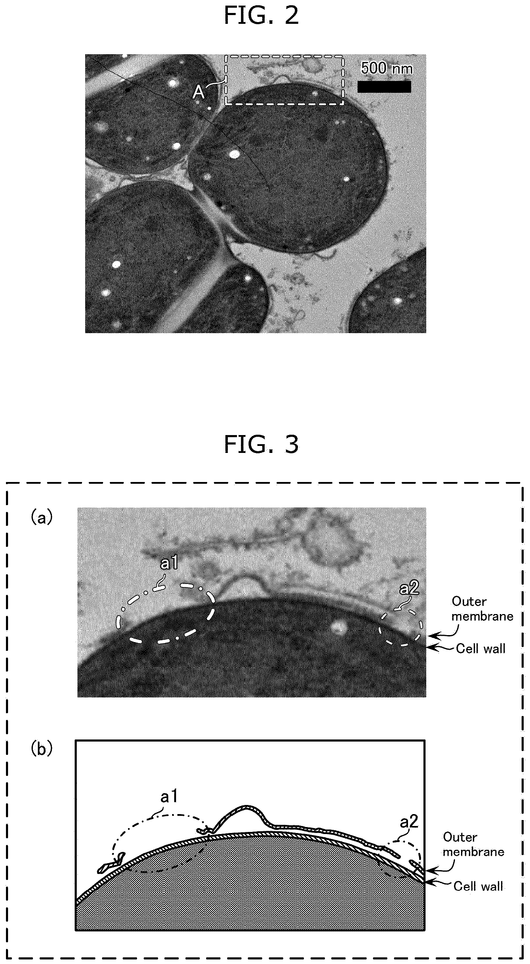

   FIG. 2 is a transmission electron microscope observation image of an ultrathin section of a modified cyanobacterium of Example 1.
[FIG. 3]
   FIG. 3 is an enlarged image of broken line region A of FIG. 2.
[FIG. 4]

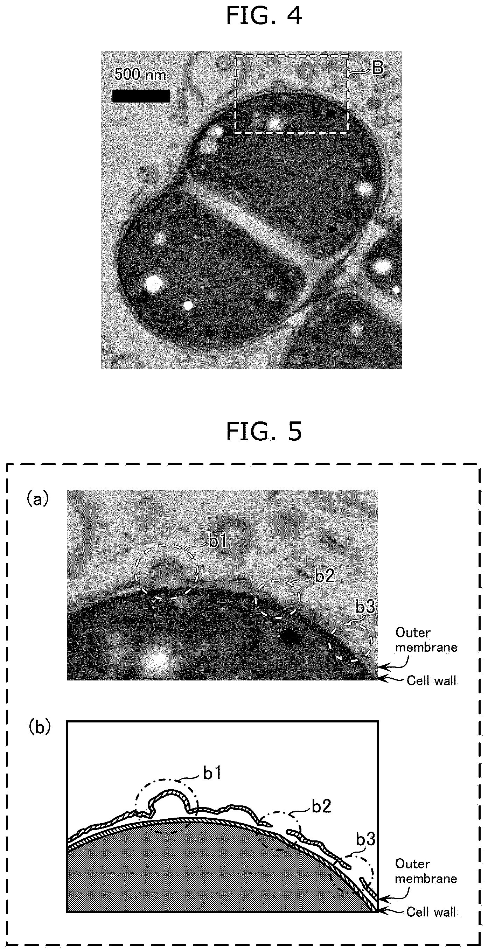

   FIG. 4 is a transmission electron microscope image of an ultrathin section of a modified cyanobacterium of Example 2.
[FIG. 5]
   FIG. 5 is an enlarged image of broken line region B of FIG. 4.
[FIG. 6]

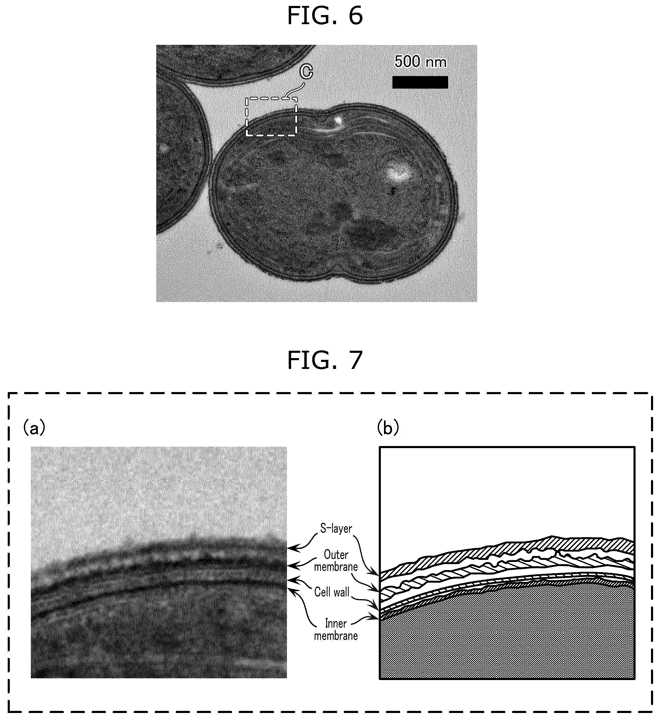

   FIG. 6 is a transmission electron microscope image of an ultrathin section of a modified cyanobacterium of Comparative Example 1.
[FIG. 7]
   FIG. 7 is an enlarged view of broken line region C of FIG. 6.
[FIG. 8]

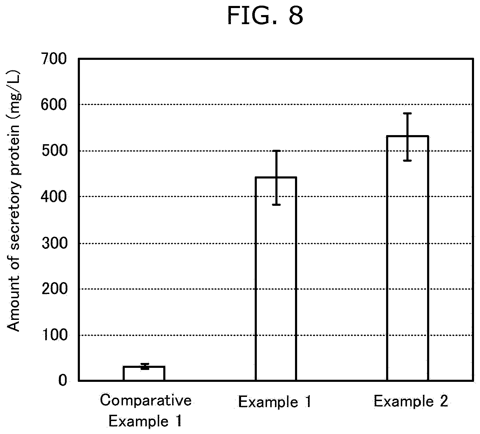

   FIG. 8 is a graph illustrating the amount of protein (n = 3, error bar = SD) in the culture supernatant of a modified cyanobacterium in Example 1, Example 2, and Comparative Example 1.

### [Description of Embodiments]

### (Underlying knowledge forming basis of present disclosure)

Cyanobacterium (also called blue-green bacterium or blue-green alga), a group of eubacterium, produces oxygen by splitting water through photosynthesis, and fixes CO₂ in air. Cyanobacterium can also fix nitrogen (N₂) in air, depending on its species. Rapid growth and high light use efficiency are known as characteristics of cyanobacterium. In addition, its genetic manipulation is easier than that of other eukaryotic alga species. Therefore, the utilization of cyanobacterium among the photosynthetic microbes is under active research and development. As mentioned above, the production of fuel such as ethanol (NPL 1), isobutanol (NPL 2), alkanes (PTL 2), and fatty acid (PTL 1) has been reported as examples of bio-manufacturing using cyanobacterium.

The production of materials serving as nutrient sources for organisms using cyanobacterium is also under research and development. For example, since protein can be synthesized only by living organisms, photosynthetic microbes which utilize light energy and CO₂ in the atmosphere are considered useful as an organism species for use in convenient and efficient protein production.

For example, NPL 3 describes a protein production method using a cyanobacterium *Synechocystis* sp. PCC6803 strain. The literature discloses the nucleotide sequence of a promoter for activating the gene transcription of an ethylene-forming enzyme with high efficiency, and a ribosomal binding sequence for enhancing translation.

NPL 4 discloses, in the case of expressing a recombinant protein with a *Synechocystis* sp. PCC6803 strain as a host, an approach for inserting a gene encoding the protein to a NS-pCC2 region on a plasmid retained by this strain. Use of the approach has been reported to improve the expression efficiency of the recombinant protein by approximately 14 times as compared with the case of inserting the gene onto chromosomal DNA (deoxyribonucleic acid).

However, the conventional techniques mentioned above require disrupting the bacterial cell of the PCC6803 strain for retrieving protein produced within the bacterial cell of the PCC6803 strain. Hence, the retrieval of the protein is time-consuming. Since various materials exist within the bacterial cell, it may be necessary to remove these materials and purify the protein of interest. Hence, the protein yield is decreased. It is also necessary to provide a fresh bacterial strain for each protein production, which is time-consuming and increases production cost. Thus, the conventional techniques mentioned above still have a low level of efficiency of protein production using cyanobacterium. There is a demand for the development of technology with higher production efficiency.

Accordingly, the present inventor has found that protein produced within the bacterial cell of cyanobacterium is easily secreted to the outside of the bacterial cell by partially detaching the outer membrane which surrounds the cell wall of cyanobacterium from the cell wall. As a result, the protein secreted to the outside of the bacterial cell can be efficiently retrieved without disrupting the bacterial cell of the cyanobacterium. Furthermore, production efficiency is improved because the cyanobacterium can be continuously used even after protein retrieval.

Therefore, the present disclosure can provide a modified cyanobacterium having improved protein secretory productivity. Furthermore, by culturing the modified cyanobacterium according to the present disclosure, protein can be efficiently produced.

### (Outline of present disclosure)

An outline of an aspect of the present disclosure is as described below.

A modified cyanobacterium according to an aspect of the present disclosure is a modified cyanobacterium in which a function of a protein involved in binding between an outer membrane and a cell wall of cyanobacterium is suppressed or lost.

As a result, the binding (e.g., binding level and binding force) between the cell wall and the outer membrane is partially reduced in the modified cyanobacterium. This facilitates partially detaching the outer membrane from the cell wall. Hence, protein produced within the bacterial cell easily leaks out to the outside of the outer membrane, i.e., the outside of the bacterial cell. Thus, the modified cyanobacterium according to an aspect of the present disclosure can provide cyanobacterium having improved secretory productivity of protein. Furthermore, the modified cyanobacterium according to an aspect of the present disclosure eliminates the need of retrieving protein by disrupting the bacterial cell and can therefore be repeatedly used in protein production even after protein retrieval.

For example, in the modified cyanobacterium according to an aspect of the present disclosure, the protein involved in the binding between the outer membrane and the cell wall may be at least one of a surface layer homology (SLH) domain-containing outer membrane protein or a cell wall-pyruvic acid modifying enzyme.

As a result, in the modified cyanobacterium, for example, (i) a function of at least one of a SLH domain-containing outer membrane protein which binds to the cell wall and an enzyme that catalyzes reaction to modify a linked sugar chain on the surface of the cell wall with pyruvic acid (i.e., a cell wall-pyruvic acid modifying enzyme) is suppressed or lost, or (ii) the expression of at least one of the SLH domain-containing outer membrane protein or the cell wall-pyruvic acid modifying enzyme is suppressed. Hence, the binding (i.e., binding level and binding force) between the SLH domain of the SLH domain-containing outer membrane protein in the outer membrane and a covalently linked sugar chain on the surface of the cell wall is reduced. Accordingly, the outer membrane is easily detached from the cell wall at a site having the weakened binding between the outer membrane and the cell wall. Thus, according to the modified cyanobacterium according to an aspect of the present disclosure, protein produced within the bacterial cell easily leaks out to the outside of the bacterial cell because the outer membrane is easy to partially detach from the cell wall due to the weakened binding between the cell wall and the outer membrane.

For example, in the modified cyanobacterium according to an aspect of the present disclosure, the SLH domain-containing outer membrane protein may be: SIr1841 having an amino acid sequence represented by SEQ ID NO: 1; NIES970_09470 having an amino acid sequence represented by SEQ ID NO: 2; Anacy_3458 having an amino acid sequence represented by SEQ ID NO: 3; or a protein having an amino acid sequence that is at least 50 percent identical to the amino acid sequence of any one of the SIr1841, the NIES970_09470, and the Anacy_3458.

As a result, in the modified cyanobacterium, for example, (i) the function of the SLH domain-containing outer membrane protein represented by any one of SEQ ID NOs: 1 to 3 or a protein having an amino acid sequence that is at least 50 percent identical to the amino acid sequence of any one of these SLH domain-containing outer membrane proteins is suppressed or lost, or (ii) the expression of the SLH domain-containing outer membrane protein represented by any one of SEQ ID NOs: 1 to 3 or a protein having an amino acid sequence that is at least 50 percent identical to the amino acid sequence of any one of these SLH domain-containing outer membrane proteins is suppressed. Hence, in the modified cyanobacterium, (i) the function of the SLH domain-containing outer membrane protein or a protein functionally equivalent to the SLH domain-containing outer membrane protein in the outer membrane is suppressed or lost, or (ii) the expression level of the SLH domain-containing outer membrane protein or a protein functionally equivalent to the SLH domain-containing outer membrane protein in the outer membrane is decreased. Therefore, according to the modified cyanobacterium according to an aspect of the present disclosure, the binding level and binding force with which a binding domain (e.g., the SLH domain) for binding the outer membrane with the cell wall binds to the cell wall are reduced. This facilitates partially detaching the outer membrane from the cell wall.

For example, in the modified cyanobacterium according to an aspect of the present disclosure, the cell wall-pyruvic acid modifying enzyme may be: SIr0688 having an amino acid sequence represented by SEQ ID NO: 4; Synpcc7942_1529 having an amino acid sequence represented by SEQ ID NO: 5; Anacy_1623 having an amino acid sequence represented by SEQ ID NO: 6; or a protein having an amino acid sequence that is at least 50 percent identical to the amino acid sequence of any one of the Slr0688, the Synpcc7942_1529, and the Anacy_1623.

As a result, in the modified cyanobacterium, for example, (i) the function of the cell wall-pyruvic acid modifying enzyme represented by any one of SEQ ID NOs: 4 to 6 or a protein having an amino acid sequence that is at least 50 percent identical to the amino acid sequence of any one of these cell wall-pyruvic acid modifying enzymes is suppressed or lost, or (ii) the expression of the cell wall-pyruvic acid modifying enzyme represented by any one of SEQ ID NOs: 4 to 6 or a protein having an amino acid sequence that is at least 50 percent identical to the amino acid sequence of any one of these cell wall-pyruvic acid modifying enzymes is suppressed. Hence, in the modified cyanobacterium, (i) the function of the cell wall-pyruvic acid modifying enzyme or a protein functionally equivalent to the enzyme is suppressed or lost, or ii) the expression level of the cell wall-pyruvic acid modifying enzyme or a protein functionally equivalent to the enzyme is decreased. A covalently linked sugar chain on the surface of the cell wall is thereby less susceptible to modification with pyruvic acid, so that binding level and binding force of the sugar chain of the cell wall that binds to the SLH domain of the SLH domain-containing outer membrane protein in the outer membrane are reduced. Therefore, in the modified cyanobacterium according to an aspect of the present disclosure, a covalently linked sugar chain on the surface of the cell wall is less susceptible to modification with pyruvic acid, so that binding force between the cell wall and the outer membrane is weakened. This facilitates partially detaching the outer membrane from the cell wall.

For example, in the modified cyanobacterium according to an aspect of the present disclosure, a gene which causes expression of the protein involved in the binding between the outer membrane and the cell wall may be deleted or inactivated.

Accordingly, in the modified cyanobacterium, the expression of the protein involved in the binding between the cell wall and the outer membrane is suppressed, or the function of the protein is suppressed or lost. Therefore, the binding (i.e., binding level and binding force) between the cell wall and the outer membrane is partially reduced. As a result, in the modified cyanobacterium, the outer membrane is easy to partially detach from the cell wall, so that protein produced within the bacterial cell easily leaks out to the outside of the outer membrane, i.e., the outside of the bacterial cell. Thus, the modified cyanobacterium according to an aspect of the present disclosure has improved secretory productivity of protein. Furthermore, the modified cyanobacterium according to an aspect of the present disclosure eliminates the need of retrieving protein by disrupting the bacterial cell and can therefore be repeatedly used in protein production even after protein retrieval.

For example, in the modified cyanobacterium according to an aspect of the present disclosure, the gene which causes expression of the protein involved in the binding between the outer membrane and the cell wall may be at least one of a gene encoding an SLH domain-containing outer membrane protein or a gene encoding a cell wall-pyruvic acid modifying enzyme.

Accordingly, in the modified cyanobacterium, at least one of the gene encoding the SLH domain-containing outer membrane protein and the gene encoding the cell wall-pyruvic acid modifying enzyme is deleted or inactivated. Hence, in the modified cyanobacterium, for example, (i) the expression of at least one of the SLH domain-containing outer membrane protein or the cell wall-pyruvic acid modifying enzyme is suppressed, or (ii) the function of at least one of the SLH domain-containing outer membrane protein or the cell wall-pyruvic acid modifying enzyme is suppressed or lost. Hence, the binding (i.e., binding level and binding force) between the SLH domain of the SLH domain-containing outer membrane protein in the outer membrane and a covalently linked sugar chain on the surface of the cell wall is reduced. Accordingly, the outer membrane is easily detached from the cell wall at a site having the weakened binding between the outer membrane and the cell wall. Thus, according to the modified cyanobacterium according to an aspect of the present disclosure, protein produced within the bacterial cell easily leaks out to the outside of the bacterial cell because the outer membrane is easy to partially detach from the cell wall due to the weakened binding between the cell wall and the outer membrane.

For example, in the modified cyanobacterium according to an aspect of the present disclosure, the gene encoding the SLH domain-containing outer membrane protein may be: slr1841 having a nucleotide sequence represented by SEQ ID NO: 7; nies970_09470 having a nucleotide sequence represented by SEQ ID NO: 8; anacy_3458 having a nucleotide sequence represented by SEQ ID NO: 9; or a gene having a nucleotide sequence that is at least 50 percent identical to the nucleotide sequence of any one of the slr1841, the nies970_09470, and the anacy_3458.

As a result, in the modified cyanobacterium, the gene encoding the SLH domain-containing outer membrane protein, represented by any one of SEQ ID NOs: 7 to 9 or a gene having a nucleotide sequence that is at least 50 percent identical to the nucleotide sequence of any one of these genes is deleted or inactivated. Hence, in the modified cyanobacterium, (i) the expression of any one of the SLH domain-containing outer membrane proteins described above or a protein functionally equivalent to any one of these proteins is suppressed, or (i) the function of any one of the SLH domain-containing outer membrane proteins described above or a protein functionally equivalent to any one of these proteins is suppressed or lost. Thus, according to the modified cyanobacterium according to an aspect of the present disclosure, the binding level and binding force of a binding domain (e.g., the SLH domain) of the outer membrane that binds to the cell wall are reduced. This facilitates partially detaching the outer membrane from the cell wall.

For example, in the modified cyanobacterium according to an aspect of the present disclosure, the gene encoding the cell wall-pyruvic acid modifying enzyme may be: slr0688 having a nucleotide sequence represented by SEQ ID NO: 10; synpcc7942_1529 having a nucleotide sequence represented by SEQ ID NO: 11; anacy_1623 having a nucleotide sequence represented by SEQ ID NO: 12; or a gene having a nucleotide sequence that is at least 50 percent identical to the nucleotide sequence of any one of the slr0688, the synpcc7942_1529, and the anacy_1623.

As a result, in the modified cyanobacterium, the gene encoding the cell wall-pyruvic acid modifying enzyme, represented by any one of SEQ ID NOs: 10 to 12 or a gene having a nucleotide sequence that is at least 50 percent identical to the nucleotide sequence of any one of these enzyme-encoding genes is deleted or inactivated. Hence, in the modified cyanobacterium, (i) the expression of any one of the cell wall-pyruvic acid modifying enzymes described above or a protein functionally equivalent to any one of these enzymes is suppressed, or (ii) the function of any one of the cell wall-pyruvic acid modifying enzymes described above or a protein functionally equivalent to any one of these enzymes is suppressed or lost. A covalently linked sugar chain on the surface of the cell wall is thereby less susceptible to modification with pyruvic acid, so that binding level and binding force of the sugar chain of the cell wall that binds to the SLH domain of the SLH domain-containing outer membrane protein in the outer membrane are reduced. Thus, in the modified cyanobacterium according to an aspect of the present disclosure, a decreased amount of a sugar chain on cell wall that binds to the outer membrane is modified with pyruvic acid, so that binding force between the cell wall and the outer membrane is weakened. This facilitates partially detaching the outer membrane from the cell wall.

Furthermore, a modified cyanobacterium production method according to an aspect of the present disclosure includes: causing a function of a protein involved in binding between an outer membrane and a cell wall of cyanobacterium to be suppressed or lost.

As a result, the binding (e.g., binding level and binding force) between the cell wall and the outer membrane is partially reduced in the produced modified cyanobacterium. This facilitates partially detaching the outer membrane from the cell wall. Hence, in the modified cyanobacterium, protein produced within the bacterial cell easily leaks out to the outside of the outer membrane (i.e., the outside of the bacterial cell). Thus, the modified cyanobacterium production method according to an aspect of the present disclosure can provide cyanobacterium having improved secretory productivity of protein. Furthermore, the produced modified cyanobacterium eliminates the need of disrupting the bacterial cell for protein retrieval because protein produced within the bacterial cell easily leaks out to the outside of the bacterial cell. Thus, the modified cyanobacterium production method according to an aspect of the present disclosure can provide a modified cyanobacterium with high use efficiency that can be repeatedly used even after protein retrieval.

Furthermore, a protein production method according to an aspect of the present disclosure includes culturing any one of the above-described modified cyanobacterium.

As a result, the modified cyanobacterium enables protein that has leaked out to the outside of the bacterial cell to be retrieved and therefore eliminates the need of retrieving the bacterial cell from a culture solution and disrupting the bacterial cell for protein retrieval. While the modified cyanobacterium is cultured, the protein can be retrieved. Furthermore, the modified cyanobacterium can be repeatedly used even after protein retrieval and therefore eliminates the need of providing a fresh modified cyanobacterium for each protein production. Thus, the protein production method according to an aspect of the present disclosure can efficiently produce protein.

Hereinafter, embodiments will be described in detail with reference to the drawings.

It should be noted that each of the subsequently described embodiments shows a generic or specific example. Numerical values, materials, steps, the processing order of the steps indicated in the following embodiments are merely examples, and thus are not intended to limit the present disclosure. Furthermore, among the elements in the following embodiments, elements that are not described in the independent claims indicating the broadest concepts are described as optional elements.

Furthermore, the figures are not necessarily precise illustrations. In the figures, elements that are substantially the same are given the same reference signs, and overlapping description thereof may be omitted or simplified.

Moreover, in the following embodiments, numerical ranges include, not only the precise meanings, but also substantially equal ranges, such as, for example, a measured amount (for example, the number, the concentration, etc.) of a protein or a range thereof, etc.

In the present specification, both of a bacterial cell and a cell refer to one individual of cyanobacterium.

### [Embodiment]

### [1. Definitions]

In the present specification, the identity of a nucleotide sequence or an amino acid sequence is calculated with Basic Local Alignment Search Tool (BLAST) algorithm. Specifically, the identity is calculated by pairwise analysis with the BLAST program available in the website of the National Center for Biotechnology Information (NCBI) (https://blast.ncbi.nlm.nih.gov/Blast.cgi). Information on cyanobacterium genes and proteins encoded by these genes are published in, for example, the NCBI database mentioned above and Cyanobase (http://genome.microbedb.jp/cyanobase/). The amino acid sequence of the protein of interest and the nucleotide sequence of a gene encoding the protein can be obtained from these databases.

Cyanobacterium, also called blue-green alga or blue-green bacterium, is a group of prokaryote that collects light energy through chlorophyll and performs photosynthesis while generating oxygen through the splitting of water using the obtained energy. Cyanobacterium is highly diverse and includes, for example, unicellular species such as *Synechocystis* sp. PCC 6803 and filamentous species having multicellular filaments such as *Anabaena* sp. PCC 7120, in terms of cell shape. There are also thermophilic species such as *Thermosynechococcus elongatus,* marine species such as *Synechococcus elongatus,* and freshwater species such as *Synechocystis,* in terms of growth environment. Other examples thereof include many species having unique features, including species, such as *Microcystis aeruginosa,* which have a gas vesicle and produce toxin, and *Gloeobacter violaceus* which lacks thylakoid and has a light-harvesting antenna protein called phycobilisome in the plasma membrane.

FIG. 1 is a diagram schematically illustrating a cell surface of a cyanobacterium. As illustrated in FIG. 1, the cell surface of cyanobacterium is constituted by a plasma membrane (also referred to as inner membrane 1), peptidoglycan 2, and outer membrane 5 which is a lipid membrane that forms the outermost layer of the cell, in order from the inside. Sugar chain 3 constituted by glucosamine and mannosamine, etc. is covalently linked to peptidoglycan 2, and pyruvic acid is bound with this covalently linked sugar chain 3 (NPL 5: Jurgens and Weckesser, 1986, J. Bacteriol., 168: 568-573). In the present specification, peptidoglycan 2 and covalently linked sugar chain 3 are collectively referred to as cell wall 4. The space between the plasma membrane (i.e., inner membrane 1) and outer membrane 5 is called periplasm where various enzymes involved in protein degradation or conformation formation, lipid or nucleic acid degradation, or uptake of extracellular nutrients, etc. are present.

A SLH domain-containing outer membrane protein (e.g., SIr1841 in the figure) has a C-terminal region embedded in a lipid membrane (also referred to as outer membrane 5) and N-terminal SLH domain 7 projecting from the lipid membrane, and is widely distributed in cyanobacterium and bacteria belonging to the class *Negativicutes,* a group of Gram-negative bacteria (NPL 6: Kojima et al., 2016, Biosci. Biotech. Biochem., 10: 1954-1959). The region embedded in the lipid membrane (i.e., outer membrane 5) forms a channel that allows hydrophilic materials to permeate the outer membrane, whereas SLH domain 7 has a function of binding to cell wall 4 (NPL 7: Kowata et al., 2017, J. Bacteriol., 199: e00371-17). The binding of SLH domain 7 to cell wall 4 requires modifying covalently linked sugar chain 3 on peptidoglycan 2 with pyruvic acid (Kojima et al., 2016, J. Biol. Chem., 291: 20198-20209). Examples of the gene encoding SLH domain-containing outer membrane protein 6 include slr1841 and sIr1908 retained by *Synechocystis* sp. PCC 6803, and oprB retained by *Anabaena* sp. 90.

An enzyme that catalyzes the pyruvic acid modification reaction of covalently linked sugar chain 3 (hereinafter, referred to as cell wall-pyruvic acid modifying enzyme 9) in peptidoglycan 2 was identified in a Gram-positive bacterium *Bacillus anthracis* and designated as CsaB (NPL 8: Mesnage et al., 2000, EMBO J., 19: 4473-4484). Many species of cyanobacterium whose genomic nucleotide sequence is published retains a gene encoding a homologous protein having an amino acid sequence that has 30% or higher identity to the amino acid sequence of CsaB. Examples thereof include slr0688 retained by *Synechocystis* sp. PCC 6803 and synpcc7502_03092 retained by *Synechococcus* sp. 7502.

In cyanobacterium, CO₂ fixed by photosynthesis is converted to various amino acids through multiple stages of enzymatic reaction. Protein is synthesized in the cytoplasm of cyanobacterium with these amino acids as starting materials. Such protein includes protein that functions in the cytoplasm and protein that is transported from the cytoplasm to the periplasm and functions in the periplasm. However, any case where protein is actively secreted to the outside of the cell has not been reported on cyanobacterium so far.

Cyanobacterium has high photosynthetic ability and therefore need not necessarily to take up organic substances as nutrients from the outside. Hence, cyanobacterium has only a very small amount of a channel protein, such as organic channel protein 8 (e.g., SIr1270) of FIG. 1, which permits permeation of organic substances, in outer membrane 5. For example, *Synechocystis* sp. PCC 6803 has only approximately 4% of organic channel protein 8 which permits permeation of organic substances based on the amount of total protein in outer membrane 5. On the other hand, outer membrane 5 of cyanobacterium is rich in an ion channel protein, such as SLH domain-containing outer membrane protein 6 (e.g., SIr1841) of FIG. 1, which permits selective permeation of only inorganic ions, for high-efficiency cellular uptake of inorganic ions necessary for growth. For example, in *Synechocystis* sp. PCC 6803, the ion channel protein which permits permeation of inorganic ions accounts for approximately 80% of the total protein of outer membrane 5.

Thus, cyanobacterium is considered to have the difficulty in actively secreting protein produced within the bacterial cell to the outside of the bacterial cell, due to very few channels which permit permeation of organic substances such as protein in outer membrane 5.

### [2. Modified cyanobacterium]

Subsequently, the modified cyanobacterium according to the present embodiment will be described with reference to FIG. 1.

In the modified cyanobacterium according to the present embodiment, a function of a protein involved in binding between outer membrane 5 and cell wall 4 (hereinafter, also referred to as a binding-related protein) of cyanobacterium is suppressed or lost. As a result, the binding (e.g., binding level and binding force) between outer membrane 5 and cell wall 4 is partially reduced in the modified cyanobacterium. This facilitates partially detaching outer membrane 5 from cell wall 4. Hence, the modified cyanobacterium has improved secretory productivity of protein that is produced within the bacterial cell and secreted to the outside of the bacterial cell. Furthermore, the modified cyanobacterium eliminates the need of retrieving protein by disrupting the bacterial cell and can therefore be repeatedly used in protein production even after protein retrieval. In the present specification, to make protein within the bacterial cell by the modified cyanobacterium is referred to as production, and to secrete the produced protein to the outside of the bacterial cell is referred to as secretory production.

The protein involved in binding between outer membrane 5 and cell wall 4 may be at least one of SLH domain-containing outer membrane protein 6 or cell wall-pyruvic acid modifying enzyme 9. In the present embodiment, in the modified cyanobacterium, for example, the function of at least one of SLH domain-containing outer membrane protein 6 or cell wall-pyruvic acid modifying enzyme 9 is suppressed or lost. For example, in the modified cyanobacterium, (i) the function of at least one of SLH domain-containing outer membrane protein 6 or cell wall-pyruvic acid modifying enzyme 9 may be suppressed or lost, or (i) at least one of the expression of SLH domain-containing outer membrane protein 6 which binds to cell wall 4 or an enzyme that catalyzes the pyruvic acid modification reaction of a linked sugar chain on the surface of cell wall 4 (i.e., cell wall-pyruvic acid modifying enzyme 9) may be suppressed. As a result, the binding (e.g., binding level and binding force) between SLH domain 7 of SLH domain-containing outer membrane protein 6 in outer membrane 5 and covalently linked sugar chain 3 on the surface of cell wall 4 is reduced. Hence, outer membrane 5 is easily detached from cell wall 4 at a site having the weakened binding therebetween. Owing to partial detachment of outer membrane 5 from cell wall 4, protein present in the cell, particularly, the periplasm, of the modified cyanobacterium easily leaks out to the outside of the cell (outside of outer membrane 5).

As mentioned above, cyanobacterium has high photosynthetic ability and therefore need not necessarily to take up organic substances as nutrients from the outside. Hence, light, air, water and trace inorganic substances suffice for cyanobacterium culture. Cyanobacterium takes up inorganic substances into the cell through the ion channel of outer membrane 5 and intracellularly produces protein. Various proteins exist, particularly, in the periplasm of the space between outer membrane 5 and cell wall 4. In the modified cyanobacterium according to the present embodiment, a function of a protein involved in binding between outer membrane 5 and cell wall 4 is suppressed or lost. This facilitates partially detaching outer membrane 5 from cell wall 4. A periplasmic protein leaks out into a culture solution from a site where outer membrane 5 is detached. As a result, the modified cyanobacterium has improved secretory productivity of protein that is produced within the bacterial cell and secreted to the outside of the bacterial cell.

Hereinafter, a cyanobacterium modified so as to partially detach outer membrane 5 from cell wall 4 by suppressing a function of at least one binding-related protein of SLH domain-containing outer membrane protein 6 and cell wall-pyruvic acid modifying enzyme 9 will be specifically described.

The type of the cyanobacterium before at least one of the expression of SLH domain-containing outer membrane protein 6 or the expression of cell wall-pyruvic acid modifying enzyme 9 is suppressed or lost (hereinafter, referred to as a parent cyanobacterium), which serves as the parent microbe of the modified cyanobacterium according to the present embodiment, is not particularly limited and may be any type of cyanobacterium. The parent cyanobacterium may be, for example, the genus *Synechocystis, Synechococcus, Anabaena,* or *Thermosynechococcus,* and may be *Synechocystis* sp. PCC 6803, *Synechococcus* sp. PCC 7942, or *Thermosynechococcus elongatus* BP-1 among them.

The amino acid sequences of SLH domain-containing outer membrane protein 6 and the enzyme that catalyzes the pyruvic acid modification reaction of the cell wall (i.e., cell wall-pyruvic acid modifying enzyme 9) in the parent cyanobacterium, the nucleotide sequences of genes encoding these binding-related proteins, and the positions of the genes on chromosomal DNA or a plasmid can be confirmed in the NCBI database and Cyanobase mentioned above.

SLH domain-containing outer membrane protein 6 or cell wall-pyruvic acid modifying enzyme 9, the function of which is suppressed or lost in the modified cyanobacterium according to the present embodiment may be from any parent cyanobacterium and is not limited by the location where a gene encoding it resides (e.g., on chromosomal DNA or on a plasmid) as long as the parent cyanobacterium carries it.

SLH domain-containing outer membrane protein 6 may be, for example, SIr1841, SIr1908, or SIr0042 when the parent cyanobacterium is the genus *Synechocystis,* may be NIES970_09470, etc. when the parent cyanobacterium is the genus *Synechococcus,* may be Anacy_5815 or Anacy_3458, etc. when the parent cyanobacterium is the genus *Anabaena,* may be A0A0F6U6F8_MICAE, etc. when the parent cyanobacterium is the genus *Microcystis,* may be A0A3B8XX12_9CYAN, etc. when the parent cyanobacterium is the genus *Cyanothece,* may be A0A1Q8ZE23_9CYAN, etc. when the parent cyanobacterium is the genus *Leptolyngbya,* includes A0A1Z4R6U0_9CYAN when the parent cyanobacterium is the genus *Calothrix,* may be A0A1C0VG86_9NOSO, etc. when the parent cyanobacterium is the genus *Nostoc,* may be B1WRN6_CROS5, etc. when the parent cyanobacterium is the genus *Crocosphaera,* and may be K9TAE4_9CYAN, etc. when the parent cyanobacterium is the genus *Pleurocapsa.*

More specifically, SLH domain-containing outer membrane protein 6 may be, for example, SIr1841 (SEQ ID NO: 1) of *Synechocystis* sp. PCC 6803, NIES970_09470 (SEQ ID NO: 2) of *Synechococcus* sp. NIES-970, or Anacy_3458 (SEQ ID NO: 3) of *Anabaena cylindrica* PCC 7122. Alternatively, a protein having an amino acid sequence that is at least 50 percent identical to the amino acid sequence of any one of these SLH domain-containing outer membrane proteins 6 may be used.

As a result, in the modified cyanobacterium, for example, (i) the function of SLH domain-containing outer membrane protein 6 represented by any one of SEQ ID NOs: 1 to 3 or a protein having an amino acid sequence that is at least 50 percent identical to the amino acid sequence of any one of these SLH domain-containing outer membrane proteins 6 may be suppressed or lost, or (ii) the expression of SLH domain-containing outer membrane protein 6 represented by any one of SEQ ID NOs: 1 to 3 or a protein having an amino acid sequence that is at least 50 percent identical to the amino acid sequence of any one of these SLH domain-containing outer membrane proteins 6 may be suppressed. Hence, in the modified cyanobacterium, (i) the function of SLH domain-containing outer membrane protein 6 or a protein functionally equivalent to SLH domain-containing outer membrane protein 6 in outer membrane 5 is suppressed or lost, or (ii) the expression level of SLH domain-containing outer membrane protein 6 or a protein functionally equivalent to SLH domain-containing outer membrane protein 6 in outer membrane 5 is decreased. Therefore, in the modified cyanobacterium according to the present embodiment, the binding level and binding force with which a binding domain (e.g., SLH domain 7) for binding outer membrane 5 with cell wall 4 binds to cell wall 4 are reduced. This facilitates partially detaching outer membrane 5 from cell wall 4.

In general, a protein having an amino acid sequence that is at least 30 percent identical to the amino acid sequence of a protein has high conformational homology to the protein and therefore, is reportedly likely to be functionally equivalent to the protein. Hence, SLH domain-containing outer membrane protein 6, the function of which is suppressed or lost may be, for example, a protein or a polypeptide which has an amino acid sequence that has 40% or higher, preferably 50% or higher, more preferably 60% or higher, further preferably 70% or higher, still further preferably 80% or higher, even further preferably 90% or higher identity to the amino acid sequence of SLH domain-containing outer membrane protein 6 represented by any one of SEQ ID NOs: 1 to 3, and which has a function of binding to covalently linked sugar chain 3 of cell wall 4.

Cell wall-pyruvic acid modifying enzyme 9 may be, for example, SIr0688 when the parent cyanobacterium is the genus *Synechocystis,* may be Syn7502_03092 or Synpcc7942_1529, etc. when the parent cyanobacterium is the genus *Synechococcus,* may be ANA_C20348 or Anacy_1623, etc. when the parent cyanobacterium is the genus *Anabaena,* may be CsaB (NCBI accession ID: TRU80220), etc. when the parent cyanobacterium is the genus *Microcystis,* may be CsaB (NCBI accession ID: WP_107667006.1), etc. when the parent cyanobacterium is the genus *Cyanothece,* may be CsaB (NCBI accession ID: WP_026079530.1), etc. when the parent cyanobacterium is the genus *Spirulina,* may be CsaB (NCBI accession ID: WP_096658142.1), etc. when the parent cyanobacterium is the genus *Calothrix,* may be CsaB (NCBI accession ID: WP_099068528.1), etc. when the parent cyanobacterium is the genus *Nostoc,* may be CsaB (NCBI accession ID: WP_012361697.1), etc. when the parent cyanobacterium is the genus *Crocosphaera,* and may be CsaB (NCBI accession ID: WP_036798735), etc. when the parent cyanobacterium is the genus *Pleurocapsa.*

More specifically, cell wall-pyruvic acid modifying enzyme 9 may be, for example, SIr0688 (SEQ ID NO: 4) of *Synechocystis* sp. PCC 6803, Synpcc7942_1529 (SEQ ID NO: 5) of *Synechococcus* sp. PCC 7942, or Anacy_1623 (SEQ ID NO: 6) of *Anabaena cylindrica* PCC 7122. Alternatively, a protein having an amino acid sequence that is at least 50 percent identical to the amino acid sequence of any one of these cell wall-pyruvic acid modifying enzymes 9 may be used.

As a result, in the modified cyanobacterium, for example, (i) the function of cell wall-pyruvic acid modifying enzyme 9 represented by any one of SEQ ID NOs: 4 to 6 or a protein having an amino acid sequence that is at least 50 percent identical to the amino acid sequence of any one of these cell wall-pyruvic acid modifying enzymes 9 is suppressed or lost, or (ii) the expression of cell wall-pyruvic acid modifying enzyme 9 represented by any one of SEQ ID NOs: 4 to 6 or a protein having an amino acid sequence that is at least 50 percent identical to the amino acid sequence of any one of these cell wall-pyruvic acid modifying enzymes 9 is suppressed. Hence, in the modified cyanobacterium, (i) the function of cell wall-pyruvic acid modifying enzyme 9 or a protein functionally equivalent to the enzyme is suppressed or lost, or (ii) the expression level of cell wall-pyruvic acid modifying enzyme 9 or a protein functionally equivalent to the enzyme is decreased. Covalently linked sugar chain 3 on the surface of cell wall 4 is thereby less susceptible to modification with pyruvic acid, so that binding level and binding force of sugar chain 3 of cell wall 4 that binds to SLH domain 7 of SLH domain-containing outer membrane protein 6 in outer membrane 5 are reduced. Therefore, in the modified cyanobacterium according to the present embodiment, covalently linked sugar chain 3 on the surface of cell wall 4 is less susceptible to modification with pyruvic acid, so that binding force between cell wall 4 and outer membrane 5 is weakened. This facilitates partially detaching outer membrane 5 from cell wall 4.

As mentioned above, a protein having an amino acid sequence that is at least 30 percent identical to the amino acid sequence of a protein is reportedly likely to be functionally equivalent to the protein. Hence, cell wall-pyruvic acid modifying enzyme 9, the function of which is suppressed or lost may be, for example, a protein or a polypeptide which has an amino acid sequence that has 40% or higher, preferably 50% or higher, more preferably 60% or higher, further preferably 70% or higher, still further preferably 80% or higher, even further preferably 90% or higher identity to the amino acid sequence of cell wall-pyruvic acid modifying enzyme 9 represented by any one of SEQ ID NOs: 4 to 6, and which has a function of catalyzing reaction to modify covalently linked sugar chain 3 on peptidoglycan 2 of cell wall 4 with pyruvic acid.

In the present specification, the phrase "a function of SLH domain-containing outer membrane protein 6 is suppressed or lost" means that the ability of the protein to bind to cell wall 4 is suppressed or lost, the transport of the protein to outer membrane 5 is suppressed or lost, or the ability of the protein to be embedded so as to function in outer membrane 5 is suppressed or lost.

The phrase "a function of cell wall-pyruvic acid modifying enzyme 9 is suppressed or lost" means that the function of modifying covalently linked sugar chain 3 of cell wall 4 with pyruvic acid by the protein is suppressed or lost.

An approach for suppressing or losing the functions of these proteins is not particularly limited as long as the approach is usually used for suppressing or losing protein functions. The approach may involve, for example, deleting or inactivating a gene encoding SLH domain-containing outer membrane protein 6 and a gene encoding cell wall-pyruvic acid modifying enzyme 9, inhibiting the transcription of these genes, inhibiting the translation of transcripts of these genes, or administrating inhibitors which specifically inhibit these proteins.

In the present embodiment, in the modified cyanobacterium, a gene which causes expression of the protein involved in binding between outer membrane 5 and cell wall 4 may be deleted or inactivated. As a result, in the modified cyanobacterium, the expression of the protein involved in the binding between cell wall 4 and outer membrane 5 is suppressed, or the function of the protein is suppressed or lost. Therefore, the binding (i.e., binding level and binding force) between cell wall 4 and outer membrane 5 is partially reduced. As a result, in the modified cyanobacterium, outer membrane 5 is easy to partially detach from cell wall 4, so that protein produced within the bacterial cell easily leaks out to the outside of outer membrane 5, i.e., the outside of the bacterial cell. Hence, the modified cyanobacterium according to the present embodiment has improved secretory productivity of protein that is produced within the bacterial cell and secreted to the outside of the bacterial cell. Furthermore, the modified cyanobacterium eliminates the need of retrieving protein by disrupting the bacterial cell and can therefore be repeatedly used in protein production even after protein retrieval.

The gene which causes expression of the protein involved in binding between outer membrane 5 and cell wall 4 may be, for example, at least one of a gene encoding SLH domain-containing outer membrane protein 6 or a gene encoding cell wall-pyruvic acid modifying enzyme 9. In the modified cyanobacterium, at least one of the gene encoding SLH domain-containing outer membrane protein 6 or the gene encoding cell wall-pyruvic acid modifying enzyme 9 is deleted or inactivated. Hence, in the modified cyanobacterium, for example, (i) the expression of at least one of SLH domain-containing outer membrane protein 6 or cell wall-pyruvic acid modifying enzyme 9 is suppressed, or (ii) the function of at least one of SLH domain-containing outer membrane protein 6 or cell wall-pyruvic acid modifying enzyme 9 is suppressed or lost. Hence, the binding (i.e., binding level and binding force) between SLH domain 7 of SLH domain-containing outer membrane protein 6 in outer membrane 5 and covalently linked sugar chain 3 on the surface of cell wall 4 is reduced. As a result, outer membrane 5 is easily detached from cell wall 4 at a site having the weakened binding between outer membrane 5 and cell wall 4. Thus, according to the modified cyanobacterium according to the present embodiment, protein produced within the bacterial cell easily leaks out to the outside of the bacterial cell because outer membrane 5 is easy to partially detach from cell wall 4 by the weakened binding between outer membrane 5 and cell wall 4.

In the present embodiment, for example, the transcription of at least one of the gene encoding SLH domain-containing outer membrane protein 6 or the gene encoding cell wall-pyruvic acid modifying enzyme 9 may be suppressed in order to suppress or lose the function of at least one of SLH domain-containing outer membrane protein 6 or cell wall-pyruvic acid modifying enzyme 9 in cyanobacterium.

The gene encoding SLH domain-containing outer membrane protein 6 may be, for example, slr1841, sIr1908, or slr0042 when the parent cyanobacterium is the genus *Synechocystis,* may be nies970_09470, etc. in the case of the genus *Synechococcus,* may be anacy_5815 or anacy_3458, etc. when the parent cyanobacterium is the genus *Anabaena,* may be A0A0F6U6F8_MICAE, etc. when the parent cyanobacterium is the genus *Microcystis,* may be A0A3B8XX12_9CYAN, etc. when the parent cyanobacterium is the genus *Cyanothece,* may be A0A1Q8ZE23_9CYAN, etc. when the parent cyanobacterium is the genus *Leptolyngbya,* may be A0A1Z4R6U0_9CYAN, etc. when the parent cyanobacterium is the genus *Calothrix,* may be A0A1C0VG86_9NOSO, etc. when the parent cyanobacterium is the genus *Nostoc,* may be B1WRN6_CROS5, etc. when the parent cyanobacterium is the genus *Crocosphaera,* and may be K9TAE4_9CYAN, etc. when the parent cyanobacterium is the genus *Pleurocapsa.* The nucleotide sequences of these genes can be obtained from the NCBI database or Cyanobase mentioned above.

More specifically, the gene encoding SLH domain-containing outer membrane protein 6 may be slr1841 (SEQ ID NO: 7) of *Synechocystis* sp. PCC 6803, nies970_09470 (SEQ ID NO: 8) of *Synechococcus* sp. NIES-970, anacy_3458 (SEQ ID NO: 9) of *Anabaena cylindrica* PCC 7122, or a gene having an amino acid sequence that is at least 50 percent identical to the amino acid sequence of any one of these genes.

As a result, in the modified cyanobacterium, the gene encoding SLH domain-containing outer membrane protein 6, represented by any one of SEQ ID NOs: 7 to 9 or a gene having a nucleotide sequence that is at least 50 percent identical to the nucleotide sequence of any one of these genes is deleted or inactivated. Hence, in the modified cyanobacterium, (i) the expression of any one of SLH domain-containing outer membrane proteins 6 described above or a protein functionally equivalent to any one of these proteins is suppressed, or (ii) the function of any one of SLH domain-containing outer membrane proteins 6 described above or a protein functionally equivalent to any one of these proteins is suppressed or lost. Therefore, in the modified cyanobacterium according to the present embodiment, the binding level and binding force of a cell wall 4 binding domain (e.g., SLH domain 7) of outer membrane 5 that binds to cell wall 4 are reduced. This facilitates partially detaching outer membrane 5 from cell wall 4.

As mentioned above, a protein having an amino acid sequence that is at least 30 percent identical to the amino acid sequence of a protein is reportedly likely to be functionally equivalent to the protein. Hence, a gene having a nucleotide sequence that is at least 30 percent identical to the nucleotide sequence of a gene encoding a protein is considered likely to cause expression of a protein functionally equivalent to the protein. Hence, the gene encoding SLH domain-containing outer membrane protein 6, the function of which is suppressed or lost may be, for example, a gene which has a nucleotide sequence that has 40% or higher, preferably 50% or higher, more preferably 60% or higher, further preferably 70% or higher, still further preferably 80% or higher, even further preferably 90% or higher identity to the nucleotide sequence of the gene encoding SLH domain-containing outer membrane protein 6 represented by any one of SEQ ID NOs: 7 to 9, and which encodes a protein or a polypeptide having a function of binding to covalently linked sugar chain 3 of cell wall 4.

The gene encoding cell wall-pyruvic acid modifying enzyme 9 may be, for example, slr0688 when the parent cyanobacterium is the genus *Synechocystis,* may be syn7502_03092 or synpcc7942_1529, etc. when the parent cyanobacterium is the genus *Synechococcus,* may be ana_C20348 or anacy_1623, etc. when the parent cyanobacterium is the genus *Anabaena,* may be csaB (NCBI accession ID: TRU80220), etc. when the parent cyanobacterium is the genus *Microcystis,* may be csaB (NCBI accession ID: WP_107667006.1), etc. when the parent cyanobacterium is the genus *Cyanothece,* may be csaB (NCBI accession ID:WP_026079530.1), etc. when the parent cyanobacterium is the genus *Spirulina,* may be csaB (NCBI accession ID:WP_096658142.1), etc. when the parent cyanobacterium is the genus *Calothrix,* may be csaB (NCBI accession ID:WP_099068528.1), etc. when the parent cyanobacterium is the genus *Nostoc,* may be csaB (NCBI accession ID: WP_012361697.1), etc. when the parent cyanobacterium is the genus *Crocosphaera,* and may be csaB (NCBI accession ID: WP_036798735), etc. when the parent cyanobacterium is the genus *Pleurocapsa.* The nucleotide sequences of these genes can be obtained from the NCBI database or Cyanobase mentioned above.

More specifically, the gene encoding cell wall-pyruvic acid modifying enzyme 9 may be slr0688 (SEQ ID NO: 10) of *Synechocystis* sp. PCC 6803, synpcc7942_1529 (SEQ ID NO: 11) of *Synechococcus* sp. PCC 7942, or anacy_1623 (SEQ ID NO: 12) of *Anabaena cylindrica* PCC 7122. Alternatively, a gene having a nucleotide sequence that is at least 50 percent identical to the nucleotide sequence of any one of these genes may be used.

As a result, in the modified cyanobacterium, the gene encoding cell wall-pyruvic acid modifying enzyme 9, represented by any one of SEQ ID NOs: 10 to 12 or a gene having a nucleotide sequence that is at least 50 percent identical to the nucleotide sequence of any one of these enzyme-encoding genes is deleted or inactivated. Hence, in the modified cyanobacterium, (i) the expression of any one of cell wall-pyruvic acid modifying enzymes 9 described above or a protein functionally equivalent to any one of these enzymes is suppressed, or (ii) the function of any one of cell wall-pyruvic acid modifying enzymes 9 described above or a protein functionally equivalent to any one of these enzymes is suppressed or lost. Covalently linked sugar chain 3 on the surface of cell wall 4 is thereby less susceptible to modification with pyruvic acid, so that binding level and binding force of sugar chain 3 of cell wall 4 that binds to SLH domain 7 of SLH domain-containing outer membrane protein 6 in outer membrane 5 are reduced. Thus, in the modified cyanobacterium according to the present embodiment, a decreased amount of sugar chain 3 on cell wall 4 that binds to outer membrane 5 is modified with pyruvic acid, so that binding force between cell wall 4 and outer membrane 5 is weakened. This facilitates partially detaching outer membrane 5 from cell wall 4.

As mentioned above, a gene having a nucleotide sequence that is at least 30 percent identical to the nucleotide sequence of a gene encoding a protein is considered likely to cause expression of a protein functionally equivalent to the protein. Hence, the gene encoding cell wall-pyruvic acid modifying enzyme 9, the function of which is suppressed or lost may be, for example, a gene which has a nucleotide sequence that has 40% or higher, preferably 50% or higher, more preferably 60% or higher, further preferably 70% or higher, still further preferably 80% or higher, even further preferably 90% or higher identity to the nucleotide sequence of the gene encoding cell wall-pyruvic acid modifying enzyme 9 represented by any one of SEQ ID NOs: 10 to 12, and which encodes a protein or a polypeptide having a function of catalyzing reaction to modify covalently linked sugar chain 3 on peptidoglycan 2 of cell wall 4 with pyruvic acid.

### [3. Modified cyanobacterium production method]

Subsequently, the modified cyanobacterium production method according to the present embodiment will be described. The modified cyanobacterium production method comprises causing a function of a protein involved in binding between outer membrane 5 and cell wall 4 of cyanobacterium to be suppressed or lost.

In the present embodiment, in the (i), the protein involved in binding between outer membrane 5 and cell wall 4 may be, for example, at least one of SLH domain-containing outer membrane protein 6 or cell wall-pyruvic acid modifying enzyme 9.

An approach for suppressing or losing the function of the protein is not particularly limited and may involve, for example, deleting or inactivating a gene encoding SLH domain-containing outer membrane protein 6 and a gene encoding cell wall-pyruvic acid modifying enzyme 9, inhibiting the transcription of these genes, inhibiting the translation of transcripts of these genes, or administrating inhibitors which specifically inhibit these proteins.

An approach for deleting or inactivating the gene may be, for example, the mutagenesis of one or more bases on the nucleotide sequence of the gene, the substitution of the nucleotide sequence by another nucleotide sequence, the insertion of another nucleotide sequence thereto, or the partial or complete deletion of the nucleotide sequence of the gene.

An approach for inhibiting the transcription of the gene may be, for example, the mutagenesis of a promoter region of the gene, the inactivation of the promoter by substitution by another nucleotide sequence or insertion of another nucleotide sequence, or CRISPR interference (NPL 9: Yao et al., ACS Synth. Biol., 2016, 5: 207-212). A specific approach for the mutagenesis or the substitution by or insertion of a nucleotide sequence may be, for example, ultraviolet irradiation, site-directed mutagenesis, or homologous recombination.

An approach for inhibiting the translation of a transcript of the gene may be, for example, RNA (ribonucleic acid) interference.

The function of the protein involved in binding between outer membrane 5 and cell wall 4 of cyanobacterium may be suppressed or lost to produce the modified cyanobacterium by use of any one of the above approaches.

As a result, the binding (e.g., binding level and binding force) between cell wall 4 and outer membrane 5 is partially reduced in the modified cyanobacterium produced by the production method according to the present embodiment. This facilitates partially detaching outer membrane 5 from cell wall 4. Hence, in the modified cyanobacterium, protein produced within the bacterial cell easily leaks out to the outside of outer membrane 5 (i.e., the outside of the bacterial cell). Thus, the modified cyanobacterium production method according to the present embodiment can provide a modified cyanobacterium having improved secretory productivity of protein.

The modified cyanobacterium produced by the production method according to the present embodiment eliminates the need of disrupting the bacterial cell for protein retrieval because protein produced within the bacterial cell easily leaks out to the outside of the bacterial cell. For example, the modified cyanobacterium can be cultured under appropriate conditions, and subsequently, protein secreted into the culture solution can be retrieved. Therefore, while the modified cyanobacterium is cultured, the protein in the culture solution may be retrieved. Hence, use of the modified cyanobacterium obtained by this production method enables efficient microbiological protein production to be carried out. Thus, the modified cyanobacterium production method according to the present embodiment can provide a modified cyanobacterium with high use efficiency that can be repeatedly used even after protein retrieval.

The modified cyanobacterium produced by the modified cyanobacterium production method according to the present embodiment secretes, for example, a protein group such as peptidase or phosphatase typically supposed to be present in the periplasm, to the outside of the cell. In the present embodiment, the modified cyanobacterium may be allowed to produce the desired protein, for example, by modifying a gene encoding a protein supposed to be produced within the cell of cyanobacterium, such as a protein group present in the periplasm, and replacing it with a gene encoding another protein. Thus, the modified cyanobacterium production method according to the present embodiment can also provide a modified cyanobacterium capable of conveniently and efficiently producing the desired protein.

### [4. Protein production method]

Subsequently, the protein production method according to the present embodiment will be described. The protein production method according to the present embodiment comprises culturing the modified cyanobacterium described above.

Cyanobacterium culture can generally be carried out on the basis of liquid culture or a modified method thereof using a BG-11 medium (see Table 2). Hence, the culture of the modified cyanobacterium may be similarly carried out. The culture period of the cyanobacterium for protein production can be a period during which protein accumulates with a high concentration under conditions where the bacterial cell has proliferated sufficiently, and may be, for example, 1 to 3 days or may be 4 to 7 days. A culture method may be, for example, aeration and agitation culture or shake culture.

The modified cyanobacterium thus cultured under the conditions described above produces protein within the bacterial cell and secretes the protein into the culture solution. In the case of retrieving the protein secreted in the culture solution, insoluble materials such as the cell (i.e., the bacterial cell) may be removed from the culture solution by the filtration or centrifugation, etc. of the culture solution to retrieve a culture supernatant. The protein production method according to the present embodiment eliminates the need of disrupting the cell for protein retrieval because protein is secreted to the outside of the cell of the modified cyanobacterium. Hence, the modified cyanobacterium remaining after protein retrieval can be repeatedly used in protein production.

The method for retrieving the protein secreted into the culture solution is not limited to the example described above. While the modified cyanobacterium is cultured, the protein in the culture solution may be retrieved. For example, a protein-permeable membrane may be used to retrieve protein that has passed through the permeable membrane. In this case, a useful microbe such as lactic acid bacterium may be cultured by using, as a nutrient source, the protein that has passed through the permeable membrane. Thus, treatment to remove the bacterial cell of the modified cyanobacterium from a culture solution is unnecessary because, while the modified cyanobacterium is cultured, protein in the culture solution can be retrieved. Hence, protein can be produced more conveniently and efficiently.

Damage and stress on the modified cyanobacterium can be reduced because bacterial cell retrieval treatment from a culture solution and bacterial cell disruption treatment are unnecessary. Hence, the secretory productivity of protein is less likely to be reduced in the modified cyanobacterium, and the modified cyanobacterium can be used for a longer time.

Thus, the protein production method using the modified cyanobacterium according to the present embodiment can conveniently and efficiently produce an enzyme for food ingredient starting material or compound production, a diagnostic or therapeutic enzyme in the medical field, or an enzyme for feed in the agricultural, fishery, and livestock fields.

### [Working examples]

Hereinafter, the modified cyanobacterium, the modified cyanobacterium production method, and the protein production method of the present disclosure will be specifically described with reference to working examples. However, the present disclosure is not limited by the following working examples by any means.

In the following working examples, two types of modified cyanobacteria were produced by suppressing the expression of slr1841 gene encoding a SLH domain-containing outer membrane protein (Example 1) and suppressing the expression of slr0688 gene encoding a cell wall-pyruvic acid modifying enzyme (Example 2) as methods for partially detaching the outer membrane of cyanobacterium from the cell wall. Then, the measurement of secretory productivity of protein and the identification of the secreted protein were performed as to these modified cyanobacteria. The cyanobacterium species used in the present working examples is *Synechocystis* sp. PCC 6803 (hereinafter, simply referred to as "cyanobacterium").

### (Working example 1)

In Example 1, a modified cyanobacterium was produced in which the expression of slr1841 gene encoding a SLH domain-containing outer membrane protein was suppressed.

### (1) Construction of modified cyanobacterium strain in which expression of slr1841 gene was suppressed

The gene expression suppression method used was CRISPR (Clustered Regularly Interspaced Short Palindromic Repeat) interference. In this method, the expression of the slr1841 gene can be suppressed by introducing a gene encoding dCas9 protein (hereinafter, referred to as dCas9 gene) and slr1841_sgRNA (single-guide ribonucleic acid) gene to the chromosomal DNA of cyanobacterium.

The mechanism of the suppression of gene expression by this method is as follows.

First, nuclease activity-deficient Cas9 protein (dCas9) and sgRNA (slr1841_sgRNA) complementarily binding to the nucleotide sequence of the slr1841 gene forms a complex.

Next, this complex recognizes the slr1841 gene on the chromosomal DNA of cyanobacterium and specifically binds to the slr1841 gene. This binding, which serves as steric hindrance, inhibits the transcription of the slr1841 gene. As a result, the expression of the slr1841 gene in the cyanobacterium is suppressed.

Hereinafter, a method for introducing each of the two genes described above to the chromosomal DNA of cyanobacterium will be specifically described.

### (1-1) Introduction of dCas9 gene

The dCas9 gene, operator gene for the expression control of the dCas9 gene, and spectinomycin resistance marker gene serving as an indicator for gene introduction were amplified by PCR (polymerase chain reaction) with the chromosomal DNA of a *Synechocystis* LY07 strain (hereinafter, also referred to as an LY07 strain) (see NPL 9) as a template using the primers psbA1-Fw (SEQ ID NO: 13) and psbA1-Rv (SEQ ID NO: 14) described in Table 1. In the LY07 strain, these three genes are inserted in a linked state in psbA1 gene on the chromosomal DNA and can therefore be amplified as one DNA fragment by PCR. Here, the obtained DNA fragment is referred to as a "psbA1::dCas9 cassette". The psbA1::dCas9 cassette was inserted to a pUC19 plasmid by use of In-Fusion PCR Cloning(R) to obtain a pUC19-dCas9 plasmid.

**[Table 1]**

| **Primer name** | **Nucleotide sequence** | **SEQ ID NO** |
|---|---|---|
| psbA1-Fw | 5' -CAGTGAATTCGAGCTCGGTATATAGCGTTGCAGTCCCTGG-3' | 13 |
| psbA1-Rv | 5' -GATTACGCCAAGCTTGCATGACCGCGGTCACTTCATAACC-3' | 14 |
| slr2030-Fw | 5' -CAGTGAATTCGAGCTCGGTAATAACCGTTGTCCCTTTTGTTTCATCG-3' | 15 |
| sgRNA_slr1841-Rv | 5' -TGTTAGTGAGCCCTGCTGTTAGCTCCCAGTATCTCTATCACTGAT-3' | 16 |
| sgRNA_slr1841-Fw | 5' -ACAGCAGGGCTCACTAACAGTTTTAGAGCTAGAAATAGCAAGTTAAAATAA-3' | 17 |
| slr2031-Rv | 5' -GATTACGCCAAGCTTGCATGGGGAACAAGCTGAATCTGGGCATC-3' | 18 |
| sgRNA_slr0688-Rv | 5' -TTTTAGTCTGTTTGCTGCATAGCTCCCAGTATCTCTATCACTGAT-3' | 19 |
| sgRNA_slr0688-Fw | 5' -TGCAGCAAACAGACTAAAAGTTTTAGAGCTAGAAATAGCAAGTTAAAATAA-3' | 20 |

1 µg of the obtained pUC19-dCas9 plasmid and a cyanobacterium culture solution (bacterial cell concentration OD730 = approximately 0.5) were mixed, and the pUC19-dCas9 plasmid was introduced into the cyanobacterium cells by spontaneous transformation. The transformed cells were selected by growth on a BG-11 agar medium containing 20 µg/mL spectinomycin. In the selected cells, homologous recombination occurred between the psbA1 gene on the chromosomal DNA and the upstream and downstream fragment regions of psbA1 on the pUC19-dCas9 plasmid. In this way, a *Synechocystis* dCas9 strain having the insert of the dCas9 cassette in the psbA1 gene region was obtained. The composition of the BG-11 medium used is as described in Table 2.

**[Table 2]**

| **Component** | **Content (mg/L)** |
|---|---|
| EDTA-Na | 1 |
| Ammonium Iron Citrate | 6 |
| NaNO₃ | 1500 |
| MgSO₄ | 75 |
| K₂HPO₄ | 39 |
| CaCl₂ | 28.6 |
| H₃BO₄ | 2.8 |
| MnCl₂ | 1.8 |
| ZnSO₄ | 0.2 |
| CuSO₄ | 0.08 |
| Na₂MoO₄ | 0.02 |
| Co(NO₃)₂ | 0.005 |
| TES-KOH (pH 7.5) | 4580 |

### (1-2) Introduction of slr1841_sgRNA gene

In the CRISPR interference, sgRNA specifically binds to a target gene by introducing a sequence of approximately 20 bases complementary to the target sequence to a region called protospacer on the sgRNA gene. The protospacer sequence used in the present working examples is described in Table 3.

**[Table 3]**

| **Protospacer target gene** | **Nucleotide sequence** | **SEQ ID NO** |
|---|---|---|
| sIr1841 | 5' -ACAGCAGGGCTCACTAACA-3' | 21 |
| sIr0688 | 5' -TGCAGCAAACAGACTAAAA-3' | 22 |

In the *Synechocystis* LY07 strain, the sgRNA gene (except for the protospacer region) and kanamycin resistance marker gene are inserted in a linked state in slr2030-slr2031 genes on the chromosomal DNA. Thus, sgRNA that specifically recognizes slr1841 (slr1841_sgRNA) can be easily obtained by adding a protospacer sequence (SEQ ID NO: 21) complementary to the slr1841 gene (SEQ ID NO: 7) to primers for use in amplifying the sgRNA gene by PCR.

First, two DNA fragments were amplified by PCR with the chromosomal DNA of the LY07 strain as a template using a set of the primers slr2030-Fw (SEQ ID NO: 15) and sgRNA_slr1841-Rv (SEQ ID NO: 16) and a set of the primers sgRNA_slr1841-Fw (SEQ ID NO: 17) and slr2031-Rv (SEQ ID NO: 18) descried in Table 1.

Subsequently, a DNA fragment (sIr2030-2031: :slr1841_sgRNA) having (i) the slr2030 gene fragment, (ii) slr1841_sgRNA, (iii) kanamycin resistance marker gene, and (iv) the slr2031 gene fragment linked in order was obtained by PCR amplification with a mixed solution of the DNA fragments described above as a template using the primers slr2030-Fw (SEQ ID NO: 15) and slr2031-Rv (SEQ ID NO: 18) described in Table 1. The sIr2030-2031::slr1841_sgRNA was inserted to a pUC19 plasmid by use of In-Fusion PCR Cloning(R) to obtain a pUC19- slr1841_sgRNA plasmid.

The pUC19-slr1841_sgRNA plasmid was introduced to the *Synechocystis* dCas9 strain in the same manner as in the (1-1), and the transformed cells were selected on a BG-11 agar medium containing 30 µg/mL kanamycin. In this way, a transformant *Synechocystis* dCas9 slr1841_sgRNA strain having the insert of slr1841_sgRNA in the slr2030-slr2031 gene on the chromosomal DNA (hereinafter, also referred to as a slr1841-suppressed strain) was obtained.

### (1-3) Suppression of slr1841 gene

The promoter sequences of the dCas9 gene and the slr1841_sgRNA gene were designed such that expression was induced in the presence of anhydrotetracycline (aTc). In the present working examples, the expression of the slr1841 gene was suppressed by adding aTc (final concentration: 1 µg/mL) into the medium.

### (Working example 2)

In Example 2, a modified cyanobacterium in which the expression of slr0688 gene encoding a cell wall-pyruvic acid modifying enzyme was suppressed was obtained by the following procedures.

### (2) Construction of modified cyanobacterium strain in which expression of slr0688 gene was suppressed

sgRNA gene containing a protospacer sequence (SEQ ID NO: 22) complementary to the slr0688 gene (SEQ ID NO: 4) was introduced to the *Synechocystis* dCas9 strain by the same procedures as in the (1-2) to obtain a *Synechocystis* dCas9 slr0688_sgRNA strain. The same conditions as in the (1-2) were used except that a set of the primers slr2030-Fw (SEQ ID NO: 15) and sgRNA_slr0688-Rv (SEQ ID NO: 19) and a set of the primers sgRNA_slr0688-Fw (SEQ ID NO: 20) and slr2031-Rv (SEQ ID NO: 18) described in Table 1 were used; and a DNA fragment (sIr2030-2031: :slr0688_sgRNA) having (i) the slr2030 gene fragment, (ii) slr0688_sgRNA, (iii) kanamycin resistance marker gene, and (iv) the slr2031 gene fragment linked in order was inserted to a pUC19 plasmid by use of In-Fusion PCR Cloning(R) to obtain a pUC19-slr0688_sgRNA plasmid.

Further, the expression of the slr0688 gene was suppressed by the same procedures as in the (1-3).

### (Comparative example 1)

In Comparative Example 1, the *Synechocystis* dCas9 strain was obtained by the same procedures as in (1-1) of Example 1.

Subsequently, the state of the cell surface was observed and a protein secretory productivity test was performed as to each of the bacterial strains obtained in Example 1, Example 2 and Comparative Example 1. Hereinafter, the details will be described.

### (3) Observation of state of cell surface of bacterial strain

The respective ultrathin sections of the modified cyanobacterium *Synechocystis* dCas9 slr1841_sgRNA strain (i.e., a slr1841-suppressed strain) obtained in Example 1, the modified cyanobacterium *Synechocystis* dCas9 slr0688_sgRNA strain (hereinafter, also referred to as a slr0688-suppressed strain) obtained in Example 2, and the modified cyanobacterium *Synechocystis* dCas9 strain (hereinafter, referred to as a control strain) obtained in Comparative Example 1 were prepared, and the state of the cell surface (in other words, an outer membrane structure) was observed under an electron microscope.

### (3-1) Culture of bacterial strain

The slr1841-suppressed strain of Example 1 was inoculated at initial bacterial cell concentration OD730 = 0.05 to a BG-11 medium containing 1 µg/mL aTc and shake-cultured for 5 days under conditions of a light quantity of 100 µmol/m2/s and 30°C. The slr0688-suppressed strain of Example 2 and the control strain of Comparative Example 1 were also cultured under the same conditions as in Example 1.

### (3-2) Preparation of ultrathin section of bacterial strain

The culture solution obtained in the (3-1) was centrifuged at 2,500 g at room temperature for 10 minutes to retrieve the cells of the slr1841-suppressed strain of Example 1. Subsequently, the cells were rapidly frozen with liquid propane of -175°C and then fixed at -80°C for 2 days using an ethanol solution containing 2% glutaraldehyde and 1% tannic acid. The cells thus fixed were dehydrated with ethanol, and the dehydrated cells were impregnated with propylene oxide and then immersed in a resin (Quetol-651) solution. Then, the resin was cured by still standing at 60°C for 48 hours to embed the cells in the resin. The cells in the resin were sliced into a thickness of 70 nm using an ultramicrotome (Ultracut) to prepare an ultrathin section. This ultrathin section was stained using 2% uranium acetate and 1% lead citrate solutions to provide a transmission electron microscopy sample of the slr1841-suppressed strain of Example 1. The slr0688-suppressed strain of Example 2 and the control strain of Comparative Example 1 were also each subjected to the same operation as above to provide transmission electron microscopy samples.

### (3-3) Observation under electron microscope

The ultrathin sections obtained in the (3-2) were observed under an accelerating voltage of 100 kV using a transmission electron microscope (JEOL JEM-1400Plus). The observation results are shown in FIGs. 2 to 7.

First, the slr1841-suppressed strain of Example 1 will be described. FIG. 2 is a TEM (transmission electron microscope) image of the slr1841-suppressed strain of Example 1. FIG. 3 is an enlarged image of broken line region A of FIG. 2. (a) in FIG. 3 is an enlarged TEM image of broken line region A of FIG. 2, and (b) in FIG. 3 is a diagram graphically depicting the enlarged TEM image of (a) in FIG. 3.

As illustrated in FIG. 2, in the slr1841-suppressed strain of Example 1, the outer membrane was partially stripped (i.e., the outer membrane partially came off) from the cell wall while the outer membrane became partially loose.

In order to confirm the more detailed state of the cell surface, broken line region A was subjected to magnifying observation. As a result, as illustrated in (a) and (b) of FIG. 3, sites where the outer membrane partially came off (dot-dash line regions a1 and a2 in the figures) were able to be confirmed. Also, a site where the outer membrane became largely loose was able to be confirmed near dot-dash line region a1. This site is a site having weakened binding between the outer membrane and the cell wall. It is considered that the outer membrane swelled outward and became loose because the culture solution infiltrated into the periplasm from the outer membrane.

Subsequently, the slr0688-suppressed strain of Example 2 will be described. FIG. 4 is a TEM image of the slr0688-suppressed strain of Example 2. FIG. 5 is an enlarged image of broken line region B of FIG. 4. (a) in FIG. 5 is an enlarged TEM image of broken line region B of FIG. 4, and (b) in FIG. 5 is a diagram graphically depicting the enlarged TEM image of (a) in FIG. 5.

As illustrated in FIG. 4, in the slr0688-suppressed strain of Example 2, the outer membrane was partially stripped from the cell wall while the outer membrane partially became loose. In the slr0688-suppressed strain, it was able to be confirmed that the outer membrane was partially detached from the cell wall.

In order to confirm the more detailed state of the cell surface, broken line region B was subjected to magnifying observation. As a result, as illustrated in (a) and (b) in FIG. 5, a site where the outer membrane became largely loose (dot-dash line region b1 in the figures) and sites where the outer membrane partially came off (dot-dash line regions b2 and b3 in the figures) were able to be confirmed. Also, a site where the outer membrane was detached from the cell wall was able to be confirmed near each of dot-dash line regions b1, b2, and b3.

Subsequently, the control strain of Comparative Example 1 will be described. FIG. 6 is a TEM image of the control strain of Comparative Example 1. FIG. 7 is an enlarged image of broken line region C of FIG. 6. (a) in FIG. 7 is an enlarged TEM image of broken line region C of FIG. 6, and (b) in FIG. 7 is a diagram graphically depicting the enlarged TEM image of (a) in FIG. 7.

As illustrated in FIG. 6, the control strain of Comparative Example 1 had an ordered cell surface where the inner membrane, the cell wall, the outer membrane, and the S-layer were kept in a state layered in order. Specifically, the control strain exhibited none of the site where the outer membrane was detached from the cell wall, the site where the outer membrane was stripped (i.e., came off) from the cell wall, and the site where the outer membrane became loose, which were found in Examples 1 and 2.

### (4) Protein secretory productivity test

The slr1841-suppressed strain of Example 1, the slr0688-suppressed strain of Example 2, and the control strain of Comparative Example 1 were each cultured, and the amount of protein secreted to the outside of the cells (hereinafter, also referred to as the amount of secretory protein) was measured. Each of the bacterial strains described above was evaluated for the secretory productivity of protein from the amount of protein in the culture solution. The secretory productivity of protein refers to the ability to produce protein by secreting intracellularly produced protein to the outside of the cells. Hereinafter, a specific method will be described.

### (4-1) Culture of bacterial strain

The slr1841-suppressed strain of Example 1 was cultured in the same manner as in the (3-1). The culture was performed three independent times. The bacterial strains of Example 2 and Comparative Example 1 were also cultured under the same conditions as in the bacterial strain of Example 1.

### (4-2) Quantification of protein secreted to outside of cell

Each culture solution obtained in the (4-1) was centrifuged at 2,500 g at room temperature for 10 minutes to obtain a culture supernatant. The obtained culture supernatant was filtered through a membrane filter having a pore size of 0.22 µm to completely remove the cells of the slr1841-suppressed strain of Example 1. The amount of total protein contained in the culture supernatant thus filtered was quantified by the BCA (bicinchoninic acid) method. This series of operations was performed as to each of the three culture solutions obtained by culture performed three independent times to determine a mean and standard deviation of the amounts of protein secreted to the outside of the cells of the slr1841-suppressed strain of Example 1. The protein in the three culture solutions were also quantified under the same conditions as above as to each of the bacterial strains of Example 2 and Comparative Example 1, and a mean and standard deviation of the amounts of protein in the three culture solutions was determined.

The results are shown in FIG. 8. FIG. 8 is a graph illustrating the amount of protein (n = 3, error bar = SD) in the culture supernatant of a modified cyanobacterium in Example 1, Example 2, and Comparative Example 1.

As illustrated in FIG. 8, the amount (mg/L) of protein secreted into the culture supernatant was improved by approximately 25 times in all the slr1841-suppressed strain of Example 1 and the slr0688-suppressed strain of Example 2 compared with the control strain of Comparative Example 1.

Although the description of data is omitted, the absorbance (730 nm) of the culture solution was measured and the amount of secretory protein per g of bacterial cell dry weight (mg protein/g cell dry weight) was calculated. As a result, the amount of secretory protein per g of bacterial cell dry weight (mg protein/g cell dry weight) was improved by approximately 36 times in all the slr1841-suppressed strain of Example 1 and the slr0688-suppressed strain of Example 2 compared with the control strain of Comparative Example 1.

Furthermore, as illustrated in FIG. 8, the amount of protein secreted into the culture supernatant was larger for the slr0688-suppressed strain of Example 2 in which the expression of the gene encoding the cell wall-pyruvic acid modifying enzyme (slr0688) was suppressed than for the slr1841-suppressed strain of Example 1 in which the expression of the gene encoding the SLH domain-containing outer membrane protein (slr1841) was suppressed. This is probably related to a larger number of covalently linked sugar chains on cell wall surface than the number of the SLH domain-containing outer membrane protein (SIr1841) in the outer membrane. Specifically, the amount of protein secreted was increased from that for the slr1841-suppressed strain of Example 1 probably because the slr0688-suppressed strain of Example 2 had smaller binding level and binding force between the outer membrane and the cell wall than those of the slr1841-suppressed strain of Example 1.

From these results, it was able to be confirmed that a function of a protein related to binding between an outer membrane and a cell wall is suppressed, whereby the binding between the outer membrane and the cell wall of cyanobacterium is partially weakened so that the outer membrane is partially detached from the cell wall. It was also able to be confirmed that the weakened binding between the outer membrane and the cell wall facilitates leaking intracellularly produced protein of cyanobacterium to the outside of the cell. Thus, the modified cyanobacterium and the production method thereof according to the present embodiment was found to largely improve the secretory productivity of protein.

### (5) Identification of secreted protein

Subsequently, the secreted protein contained in the culture supernatant obtained in the (4-2) was identified by LC-MS/MS. A method will be described below.

### (5-1) Sample preparation

Cold acetone was added in an amount of 8 times the fluid volume of the culture supernatant, and the mixture was left standing at 20°C for 2 hours and then centrifuged at 20,000 g for 15 minutes to obtain a precipitate of the protein. To this precipitate, 100 mM Tris pH 8.5 and 0.5% sodium dodecanoate (SDoD) were added, and the protein was lysed with a closed sonicator. After adjustment of the protein concentration to 1 µg/mL, dithiothreitol (DTT) (final concentration: 10 mM) was added thereto, and the mixture was left standing at 50°C for 30 minutes. Subsequently, iodoacetamide (IAA) (final concentration: 30 mM) was added thereto, and the mixture was left standing at room temperature (in the dark) for 30 minutes. In order to terminate the reaction of IAA, cysteine (final concentration: 60 mM) was added thereto, and the mixture was left standing at room temperature for 10 minutes. 400 ng of trypsin was added thereto, and the mixture was left standing overnight at 37°C to convert the protein to peptide fragments. After addition of 5% TFA (trifluoroacetic acid), the mixture was centrifuged at 15,000 g at room temperature for 10 minutes to obtain a supernatant. By this operation, SDoD was removed. The sample was desalted using a C18 spin column and then dried with a centrifugal evaporator. Then, 3% acetonitrile and 0.1% formic acid were added thereto, and the sample was lysed using a closed sonicator. The peptide concentration was adjusted to 200 ng/µL.

### (5-2) LC-MS/MS analysis

The sample obtained in the (5-1) was analyzed using an LC-MS/MS apparatus (UltiMate 3000 RSLCnano LC System) under the following conditions.
Amount of sample injected: 200 ng
Column: CAPCELL CORE MP 75 µm × 250 mm
Solvent: solvent A: 0.1% aqueous formic acid solution, solvent B: 0.1% formic acid + 80% acetonitrile
Gradient program: 8% solvent B 4 minutes after sample injection, 44% solvent B 27 minutes later, 80% solvent B 28 minutes later, and completion of measurement 34 minutes later.

### (5-3) Data analysis

The obtained data was analyzed under the following conditions to perform protein and peptide identification and the calculation of quantification values.
Software: Scaffold DIA
Database: UniProtKB/Swiss Prot database (*Synechocystis* sp. PCC 6803)
Fragmentation: HCD
Precursor Tolerance: 8 ppm
Fragment Tolerance: 10 ppm
Data Acquisition Type: Overlapping DIA
Peptide Length: 8-70
Peptide Charge: 2-8
Max Missed Cleavages: 1
Fixed Modification: Carbamidomethylation
Peptide FDR: 1% or less

Among the identified proteins, 10 types of proteins are described in Table 4 in the descending order of relative quantification values from the largest one.

**[Table 4]**

| | **Protein name** | **Uniprot Accession ID** | **Gene name** |
|---|---|---|---|
| 1 | Carboxyl-terminal protease | P73458 | prc |
| 2 | Ssr1853 protein | P72639 | ssr1853 |
| 3 | SII0314 protein | 055648 | sll0314 |
| 4 | SII0858 protein | P73742 | sl10858 |
| 5 | SII0319 protein | P74789 | sl10319 |
| 6 | General secretion pathway protein G | P73704 | hofG |
| 7 | SIr0581 protein | 055408 | sIr0581 |
| 8 | Uncharacterized **WD** repeat-containing protein sll1491 | P74598 | sII1491 |
| 9 | Iron uptake protein A2 | 055835 | futA2 |
| 10 | Plastocyanin | P21697 | petE |

All the 10 types of proteins were contained in each of the culture supernatants of the slr1841-suppressed strain of Example 1 and the slr0688-suppressed strain of Example 2. All of these proteins retained a periplasm (which refers to the space between the outer membrane and the inner membrane) localization signal. From these results, it was able to be confirmed that in the modified strains of Example 1 and Example 2, the outer membrane is partially detached from the cell wall, whereby protein in the periplasm easily leaks out to the outside of the outer membrane (i.e., to the outside of the bacterial cell). Thus, the modified cyanobacterium according to the present embodiment was found to have drastically improved secretory productivity of protein.

### (6) Discussion

In the present working examples, the modified cyanobacterium of the present disclosure was able to be confirmed to secrete protein present in the bacterial cell (here, in the periplasm) to the outside of the bacterial cell. The modified cyanobacterium of the present disclosure may be genetically modified, for example, so as to produce another protein instead of the proteins identified above (i.e., protein supposed to be produced within the bacterial cell), and is therefore capable of efficiently producing the desired protein. Cyanobacterium, because of its high photosynthetic ability, can be cultured by the addition of light, water, air, and trace inorganic substances to conveniently obtain necessary protein when needed, and therefore eliminates the need of using a complicated apparatus for protein production. Protein easily loses its function, for example, when processed into supplements. Hence, the modified cyanobacterium of the present disclosure can provide protein with its function maintained. Because of these advantages, the modified cyanobacterium of the present disclosure is expected to be applied to various fields.

From "(3-3) Observation under electron microscope", it was able to be confirmed in the modified strain in which the expression of the gene encoding the protein related to the binding between the outer membrane and the cell wall was suppressed that the outer membrane partially came off by the weakened binding between the outer membrane and the cell wall; and the outer membrane partially swelled. This is probably because the culture solution easily flowed, from the outside of the cell, through osmotic pressure into a location where the outer membrane was partially detached from the cell wall by the weakened binding between the outer membrane and the cell wall; and the outer membrane partially swelled due to the culture solution that flowed therein. Since the protein concentration of the culture solution differed largely from the protein concentration in the periplasm, it is considered that the culture solution flowed into the periplasm from the outer membrane without stopping and the outer membrane that no longer withstood the internal pressure was disrupted and came off (i.e., stripped).

Among all the proteins identified by LC-MS/MS analysis, only 10 types of proteins are described in the descending order of relative quantification values from the largest one in Table 3. "(5-3) Data analysis" states that all the 10 types of proteins described in Table 3 were contained in each of the culture supernatants of the slr1841-suppressed strain of Example 1 and the slr0688-suppressed strain of Example 2. It was also confirmed that all the other proteins identified by LC-MS/MS analysis were contained in each of the culture supernatants of Example 1 and Example 2. Further, the secreted proteins were confirmed to include protein supposed to be present in the periplasm, and protein that is transported from the cytoplasm to the periplasm and functions in the periplasm.

Although a modified cyanobacterium, a modified cyanobacterium production method, and a protein production method according to the present disclosure are described above based on the foregoing embodiments, the present discloser is not limited by the foregoing embodiments. Forms obtained by making various modifications to the foregoing embodiments that can be conceived by those skilled in the art as well as other forms realized by combining some of the components in the foregoing embodiments, for as long as they do not depart from the essence of the present disclosure, are included in the scope of the present disclosure.

In the embodiments described above, examples are described in which protein produced within the bacterial cell leaks out to the outside of the bacterial cell by suppressing or losing a function of a protein involved in binding between an outer membrane and a cell wall of cyanobacterium. However, the present disclosure is not limited thereby. For example, the binding between the outer membrane and the cell wall may be weakened or the outer membrane may be rendered fragile by applying external force to cyanobacterium. Alternatively, the outer membrane may be rendered fragile by adding an enzyme or a drug to the culture solution of cyanobacterium.

### [Industrial Applicability]

The modified cyanobacterium, the modified cyanobacterium production method, and the protein production method using the modified cyanobacterium according to the present disclosure can efficiently produce protein by culturing the modified cyanobacterium by the addition of water, light, air, and trace inorganic substances. For example, an enzyme for food ingredient starting material or compound production, a diagnostic or therapeutic enzyme in the medical field, or an enzyme for feed in the agricultural, fishery, and livestock fields can be obtained.

### [Reference Signs List]

- 1: inner membrane
- 2: peptidoglycan
- 3: sugar chain
- 4: cell wall
- 5: outer membrane
- 6: SLH domain-containing outer membrane protein
- 7: SLH domain
- 8: organic channel protein
- 9: cell wall-pyruvic acid modifying enzyme

## Claims

1. A modified cyanobacterium in which a function of a protein involved in binding between an outer membrane and a cell wall of cyanobacterium is suppressed or lost.

2. The modified cyanobacterium according to claim 1, wherein
the protein involved in the binding between the outer membrane and the cell wall is at least one of a surface layer homology (SLH) domain-containing outer membrane protein or a cell wall-pyruvic acid modifying enzyme.

3. The modified cyanobacterium according to claim 2, wherein
the SLH domain-containing outer membrane protein is:
SIr1841 having an amino acid sequence represented by SEQ ID NO: 1;
NIES970_09470 having an amino acid sequence represented by SEQ ID NO: 2;
Anacy_3458 having an amino acid sequence represented by SEQ ID NO: 3; or
a protein having an amino acid sequence that is at least 50 percent identical to the amino acid sequence of any one of the SIr1841, the NIES970_09470, and the Anacy_3458.

4. The modified cyanobacterium according to claim 2, wherein
the cell wall-pyruvic acid modifying enzyme is:
SIr0688 having an amino acid sequence represented by SEQ ID NO: 4;
Synpcc7942_1529 having an amino acid sequence represented by SEQ ID NO: 5;
Anacy_1623 having an amino acid sequence represented by SEQ ID NO: 6; or
a protein having an amino acid sequence that is at least 50 percent identical to the amino acid sequence of any one of the Slr0688, the Synpcc7942_1529, and the Anacy_1623.

5. The modified cyanobacterium according to claim 1, wherein
a gene which causes expression of the protein involved in the binding between the outer membrane and the cell wall is deleted or inactivated.

6. The modified cyanobacterium according to claim 5, wherein
the gene which causes expression of the protein involved in the binding between the outer membrane and the cell wall is at least one of a gene encoding an SLH domain-containing outer membrane protein or a gene encoding a cell wall-pyruvic acid modifying enzyme.

7. The modified cyanobacterium according to claim 6, wherein
the gene encoding the SLH domain-containing outer membrane protein is:
sIr1841 having a nucleotide sequence represented by SEQ ID NO: 7;
nies970_09470 having a nucleotide sequence represented by SEQ ID NO: 8;
anacy_3458 having a nucleotide sequence represented by SEQ ID NO: 9; or
a gene having a nucleotide sequence that is at least 50 percent identical to the nucleotide sequence of any one of the sIr1841, the nies970_09470, and the anacy_3458.

8. The modified cyanobacterium according to claim 6, wherein
the gene encoding the cell wall-pyruvic acid modifying enzyme is:
slr0688 having a nucleotide sequence represented by SEQ ID NO: 10;
synpcc7942_1529 having a nucleotide sequence represented by SEQ ID NO: 11;
anacy_1623 having a nucleotide sequence represented by SEQ ID NO: 12; or
a gene having a nucleotide sequence that is at least 50 percent identical to the nucleotide sequence of any one of the slr0688, the synpcc7942_1529, and the anacy_1623.

9. A modified cyanobacterium production method comprising:
causing a function of a protein involved in binding between an outer membrane and a cell wall of cyanobacterium to be suppressed or lost.

10. A protein production method comprising:
culturing the modified cyanobacterium according to any one of claims 1 to 8.
